# EUROPEAN PATENT APPLICATION

(11) **EP 4 840 945 A1**
(43) Date of publication of application: **18.11.2026**
(21) Application number: 25738790.2
(22) Date of filing: 08.01.2025
(51) Int. Cl.: C12N 5/10, A61K 35/17, A61K 38/17, A61K 39/395, A61K 47/68, A61P 27/02, A61P 27/14, A61P 31/04, A61P 31/12, A61P 33/00, A61P 33/04, A61P 35/00, A61P 37/06, A61P 37/08, A61P 43/00, C12N 5/0783, C12N 15/12, C12N 15/13, C12N 15/62, C12N 15/63

(54) **SURVIVOR T CELLS, SURVIVOR T CELL POPULATION, AND PHARMACEUTICAL COMPOSITION**

(30) Priority: 09.01.2024 JP 2024001532
(71) Applicant: National Cancer Center, Tokyo 104-0045 (JP); Arc Therapies Inc., Tokyo, 160-0018 (JP)
(72) Inventor: NISHIKAWA, Hiroyoshi, Tokyo 104-0045 (JP); WATANABE, Keisuke, Tokyo 104-0045 (JP)
(74) Representative: Engelhard, Markus
(86) International application number: PCT/JP2025/000412
(87) International publication number: WO 2025/150522

(57) **Abstract**

The present disclosure provides cells that are competitive with cancer cells without becoming starved or exhausted even in an environment in which glucose is depleted. Specifically, the present disclosure provides survivor T cells, which are T cells with enhanced glucose uptake ability, in which the T cells are modified to express a glucose transporter and/or have enhanced expression of a glucose transporter. A cell population containing such cells, a pharmaceutical composition, a treatment method, and the like are also provided.

## Description

### Technical Field

The present disclosure relates to survivor T cells with enhanced glucose uptake ability, a survivor T cell population, and a pharmaceutical composition containing such cells or such a cell population.

### Background Art

Cancer cells reprogram their metabolic systems to favor their own cell proliferation, actively use the glycolytic pathway, which has low efficiency in ATP production, even in the presence of oxygen, and increase glucose uptake and lactic acid production (Warburg effect).

In the tumor microenvironment, many factors that suppress metabolic activity and functions of antitumor T cells are present, and in addition to immunosuppressive factors, metabolic competition between tumors and T cells is involved in formation of an immunosuppressive environment. When T cells receive stimulation through TCR, the intracellular concentration of Ca²⁺ increases, and calcineurin is activated. NFAT, a transcription factor dephosphorylated by activation of calcineurin, translocates into the nucleus, and promotes transcription of IL-2 gene and the like through interaction with other transcription factors, leading to activation.

On the other hand, because cancer cells consume glucose in large amounts, thereby causing glucose depletion, T_{eff}-type tumor-specific T cells, which require glucose as an energy source, have a decreased cellular Ca²⁺ concentration and reduced nuclear translocation of NFAT even when they receive a signal through TCR upon infiltrating into a tumor. Therefore, the T cells become dysfunctional, and cell proliferation and cytokine production are suppressed.

In addition to glucose, when nutrients such as amino acids and fatty acids are also consumed in large amounts by cancer cells, T cells competing for those nutrients become dysfunctional. As described above, in the tumor microenvironment, the antitumor effector action of tumor-specific T cells is inhibited by a metabolic mechanism that supports active proliferation of cancer cells.

Therefore, cells that are competitive with cancer cells without becoming starved or exhausted even under such an environment are desired.

### Summary of Invention

### Solution to Problem

The present disclosure provides T cells with enhanced glucose uptake ability that function without becoming starved or exhausted even in a tumor environment, a cell population containing such cells, and a pharmaceutical composition containing such cells.

Accordingly, the present disclosure provides the following.
[Item 1] A T cell with enhanced glucose uptake ability.
[Item 2] The cell according to Item 1, in which the T cell includes a chimeric antigen receptor (CAR) or a T cell receptor (TCR).
[Item 2A] The T cell according to any one of the above items, in which the T cell includes a chimeric antigen receptor (CAR).
[Item 3] The T cell according to any one of the above items, in which the T cell has an effector function.
[Item 4] The T cell according to any one of the above items, in which the T cell is a precursor cell of an effector T cell (T_{eff}).
[Item 5] The T cell according to any one of the above items, in which the CAR is expressed in the T cell.
[Item 6] The T cell according to any one of the above items, in which the glucose transporter includes GLUT1, GLUT2, GLUT3, and GLUT4.
[Item 7] The T cell according to any one of the above items, in which the glucose transporter is GLUT3.
[Item 8] The T cell according to any one of the above items, in which the T cell is a human T cell.
[Item 9] A cell population containing T cells with enhanced glucose uptake ability, in which the T cells include cells having a property of exhibiting an effector function when introduced into a body.
[Item 10] The cell population according to any one of the above items, in which the T cells are modified to express a glucose transporter and/or have enhanced expression of a glucose transporter.
[Item 11] The cell population according to any one of the above items, in which the T cells have an effector function.
[Item 12] The cell population according to any one of the above items, in which the T cells include a chimeric antigen receptor (CAR) or a T cell receptor (TCR).
[Item 12A] The cell population according to any one of the above items, in which the T cells include a chimeric antigen receptor (CAR).
[Item 13] The cell population according to any one of the above items, in which the cell population contains T_{eff}.
[Item 14] The cell population according to any one of the above items, in which the cell population includes precursor cells of T_{eff}.
[Item 14A] The cell population according to any one of the above items, in which the cell population (or precursor cells of T_{eff}) includes at least one type of cell selected from the group consisting of Tnv, Tscm, Tcm, and Temra.
[Item 15] The cell population according to any one of the above items, in which the CAR or TCR is expressed in the T cells.
[Item 16] The cell population according to any one of the above items, in which the glucose transporter includes GLUT1, GLUT2, GLUT3, and GLUT4.
[Item 17] The cell population according to any one of the above items, in which the glucose transporter is GLUT3.
[Item 18] The cell population according to any one of the above items, in which the T cells are human T cells.
[Item 19] A pharmaceutical composition containing the T cell according to any one of the above items or the cell population according to any one of the above items.
[Item 20] The pharmaceutical composition according to any one of the above items, in which the pharmaceutical composition is for treatment or prevention of cancer, an autoimmune disease, allergy, or an infectious disease.
[Item 20A] The pharmaceutical composition according to any one of the above items, in which the pharmaceutical composition is for curing cancer.
[Item 21] A pharmaceutical composition for prevention or treatment of a disease in a subject, in which the pharmaceutical composition is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 21A] The pharmaceutical composition according to any one of the above items, in which the T cells include a chimeric antigen receptor (CAR) or a T cell receptor (TCR).
[Item 21B] The pharmaceutical composition according to any one of the above items, in which the T cells include a chimeric antigen receptor (CAR).
[Item 21C] The pharmaceutical composition according to any one of the above items, in which the disease includes cancer, an autoimmune disease, allergy, or an infectious disease.
[Item 21D] The pharmaceutical composition according to any one of the above items, in which the disease includes cancer.
[Item 22] The composition according to any one of the above items, in which the glucose transporter is GLUT3.
[Item 23] The T cell according to any one of the above items or the cell population according to any one of the above items, in which the T cell or cell population is for use as a medicament.
[Item 24] The cell or cell population according to any one of the above items, in which the cell or cell population is for treatment or prevention of cancer, an autoimmune disease, allergy, or an infectious disease.
[Item 24A] The cell or cell population according to any one of the above items, in which the cell or cell population is for curing cancer.
[Item 25] A cell or cell population for prevention or treatment of a disease in a subject, in which the cell or cell population is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 25A] The cell or cell population according to any one of the above items, in which the T cell includes a chimeric antigen receptor (CAR) or a T cell receptor (TCR).
[Item 25B] The cell or cell population according to any one of the above items, in which the T cell includes a chimeric antigen receptor (CAR).
[Item 25C] The cell or cell population according to any one of the above items, in which the disease includes cancer, an autoimmune disease, allergy, or an infectious disease.
[Item 25D] The cell or cell population according to any one of the above items, in which the disease includes cancer.
[Item 26] The cell or cell population according to any one of the above items, in which the glucose transporter is GLUT3.
[Item 27] A method for treatment or prevention in a subject, the method including a step of administering an effective amount of the T cell according to any one of the above items or the cell population according to any one of the above items to the subject in need thereof.
[Item 28] The method according to any one of the above items, in which the treatment or prevention of the subject is for treatment or prevention of cancer, an autoimmune disease, allergy, or an infectious disease.
[Item 28A] The method according to any one of the above items, in which the treatment or prevention of the subject is for curing cancer.
[Item 29] A method for prevention or treatment of a disease in a subject, the method including:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 29A] The method according to any one of the above items, in which the T cells include a chimeric antigen receptor (CAR) or a T cell receptor (TCR).
[Item 29B] The method according to any one of the above items, in which the T cells include a chimeric antigen receptor (CAR).
[Item 29C] The method according to any one of the above items, in which the disease includes cancer, an autoimmune disease, allergy, or an infectious disease.
[Item 29D] The method according to any one of the above items, in which the disease includes cancer.
[Item 30] The method according to any one of the above items, in which the glucose transporter is GLUT3.
[Item 31] Use of the T cell according to any one of the above items or the cell population according to any one of the above items for producing a medicament containing the cell or cell population.
[Item 32] The use according to any one of the above items, in which the medicament is for treatment or prevention of cancer, an autoimmune disease, allergy, or an infectious disease.
[Item 32A] The use according to any one of the above items, in which the medicament is for curing cancer.
[Item 33] The use according to any one of the above items, in which the medicament is for prevention or treatment of a disease in a subject, and the medicament is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item 33A] The use according to any one of the above items, in which the T cells include a chimeric antigen receptor (CAR) or a T cell receptor (TCR).
[Item 33B] The use according to any one of the above items, in which the T cells include a chimeric antigen receptor (CAR).
[Item 33C] The use according to any one of the above items, in which the disease includes cancer, an autoimmune disease, allergy, or an infectious disease.
[Item 33D] The use according to any one of the above items, in which the disease includes cancer.
[Item 34] The use according to any one of the above items, in which the glucose transporter is GLUT3.

The present disclosure also provides the following.
[Item B0] An effector T cell modified to express a glucose transporter and/or having enhanced expression of a glucose transporter, in which the T cell exhibits an effector function under low-glucose conditions [0.5 mM] that is at least equivalent to that under normal-glucose conditions [10 mM].
[Item B1] An effector T cell including a chimeric antigen receptor (CAR), the effector T cell being modified to express a glucose transporter and/or having enhanced expression of a glucose transporter, in which the T cell exhibits an effector function under low-glucose conditions [0.5 mM] that is at least equivalent to that under normal-glucose conditions [10 mM].
[Item B2] The T cell according to the above item, in which the CAR is expressed in the T cell.
[Item B3] The T cell according to any one of the above items, in which the glucose transporter includes GLUT1, GLUT2, GLUT3, and GLUT4.
[Item B4] A T cell including a chimeric antigen receptor [CAR], the T cell being an effector T cell modified to express a glucose transporter and/or having enhanced expression of a glucose transporter, in which the T cell exhibits an effector function under low-glucose conditions [0.5 mM] that is at least equivalent to that under normal-glucose conditions [10 mM], and the glucose transporter is GLUT3.
[Item B5] An effector T cell into which a GLUT3 gene is introduced.
[Item B6] The T cell according to any one of the above items, in which the T cell is a human T cell.
[Item B7A] A cell population containing effector T cells modified to express a glucose transporter and/or having enhanced expression of a glucose transporter, in which the T cells exhibit an effector function under low-glucose conditions [0.5 mM] that is at least equivalent to that under normal-glucose conditions [10 mM].
[Item B8] The cell population according to any one of the above items, in which the CAR is expressed in the T cells.
[Item B7] A cell population containing effector T cells that include a chimeric antigen receptor [CAR] and are modified to express a glucose transporter and/or have enhanced expression of a glucose transporter, in which the T cells exhibit an effector function under low-glucose conditions [0.5 mM] that is at least equivalent to that under normal-glucose conditions [10 mM].
[Item B8] The cell population according to any one of the above items, in which the CAR is expressed in the T cells.
[Item B9] The cell population according to any one of the above items, in which the glucose transporter includes GLUT1, GLUT2, GLUT3, and GLUT4.
[Item B10] A cell population containing effector T cells that include a chimeric antigen receptor [CAR] and are modified to express a glucose transporter and/or have enhanced expression of a glucose transporter, in which the T cells exhibit an
effector function under low-glucose conditions [0.5 mM] that is at least equivalent to that under normal-glucose conditions [10 mM], and the glucose transporter is GLUT3.
[Item B11] A cell population containing effector T cells into which a GLUT3 gene is introduced.
[Item B12] The cell population according to any one of the above items, in which the T cells are human T cells.
[Item B13] A pharmaceutical composition containing the T cell according to any one of the above items or the cell population according to any one of the above items.
[Item B14] The pharmaceutical composition according to any one of the above items, in which the pharmaceutical composition is for treatment or prevention of cancer, an autoimmune disease, allergy, or an infectious disease.
[Item B15] The pharmaceutical composition according to any one of the above items, in which the pharmaceutical composition is for curing cancer.
[Item B16] A pharmaceutical composition for prevention or treatment of a disease in a subject, in which the pharmaceutical composition is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item B17] The pharmaceutical composition according to any one of the above items, in which the T cells include a chimeric antigen receptor (CAR) or a T cell receptor (TCR).
[Item B18] The pharmaceutical composition according to any one of the above items, in which the T cells include a chimeric antigen receptor (CAR).
[Item B19] The pharmaceutical composition according to any one of the above items, in which the disease includes cancer, an autoimmune disease, allergy, or an infectious disease.
[Item B20] The pharmaceutical composition according to any one of the above items, in which the disease includes cancer.
[Item B21] The composition according to any one of the above items, in which the glucose transporter is GLUT3.
[Item B22] The T cell according to any one of the above items or the cell population according to any one of the above items, in which the T cell or cell population is for use as a medicament.
[Item B23] The cell or cell population according to any one of the above items, in which the cell or cell population is for treatment or prevention of cancer, an autoimmune disease, allergy, or an infectious disease.
[Item B24] The cell or cell population according to any one of the above items, in which the cell or cell population is for curing cancer.
[Item B25] A cell or cell population for prevention or treatment of a disease in a subject, in which the cell or cell population is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item B26] The cell or cell population according to any one of the above items, in which the T cell includes a chimeric antigen receptor (CAR) or a T cell receptor (TCR).
[Item B27] The cell or cell population according to any one of the above items, in which the T cell includes a chimeric antigen receptor (CAR).
[Item B28] The cell or cell population according to any one of the above items, in which the disease includes cancer, an autoimmune disease, allergy, or an infectious disease.
[Item B29] The cell or cell population according to any one of the above items, in which the disease includes cancer.
[Item B30] The cell or cell population according to any one of the above items, in which the glucose transporter is GLUT3.
[Item B31] A method for treatment or prevention in a subject, the method including a step of administering an effective amount of the T cell according to any one of the above items or the cell population according to any one of the above items to the subject in need thereof.
[Item B32] The method according to any one of the above items, in which the treatment or prevention of the subject is for treatment or prevention of cancer, an autoimmune disease, allergy, or an infectious disease.
[Item B33] The method according to any one of the above items, in which the treatment or prevention of the subject is for curing cancer.
[Item B34] A method for prevention or treatment of a disease in a subject, the method including:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item B35] The method according to any one of the above items, in which the T cells include a chimeric antigen receptor (CAR) or a T cell receptor (TCR).
[Item B36] The method according to any one of the above items, in which the T cells include a chimeric antigen receptor (CAR).
[Item B37] The method according to any one of the above items, in which the disease includes cancer, an autoimmune disease, allergy, or an infectious disease.
[Item B38] The method according to any one of the above items, in which the disease includes cancer.
[Item B39] The method according to any one of the above items, in which the glucose transporter is GLUT3.
[Item B40] Use of the T cell according to any one of the above items or the cell population according to any one of the above items for producing a medicament containing the cell or cell population.
[Item B41] The use according to any one of the above items, in which the medicament is for treatment or prevention of cancer, an autoimmune disease, allergy, or an infectious disease.
[Item B42] The use according to any one of the above items, in which the medicament is for curing cancer.
[Item B43] The use according to any one of the above items, in which the medicament is for prevention or treatment of a disease in a subject, and the medicament is characterized by:
   (A) collecting a value of glucose uptake ability or a value of an index related thereto in the subject;
   (B) selecting T cells with enhanced glucose uptake ability, or a cell population containing the T cells, having appropriate expression characteristics according to the index; and
   (C) administering the T cells with enhanced glucose uptake ability or the cell population containing the T cells to the subject according to an appropriate administration method and dosage.
[Item B44] The use according to any one of the above items, in which the T cells include a chimeric antigen receptor (CAR) or a T cell receptor (TCR).
[Item B45] The use according to any one of the above items, in which the T cells include a chimeric antigen receptor (CAR).
[Item B46] The use according to any one of the above items, in which the disease includes cancer, an autoimmune disease, allergy, or an infectious disease.
[Item B47] The use according to any one of the above items, in which the disease includes cancer.
[Item B48] The use according to any one of the above items, in which the glucose transporter is GLUT3.

In the present disclosure, it is intended that one or more of the above features can be provided in further combinations in addition to the specified combination. Note that still further embodiments and advantages of the present disclosure will be appreciated by those skilled in the art upon reading and understanding the following detailed description, if necessary.

Note that features and remarkable operations and effects of the present disclosure other than those described above will be apparent to those skilled in the art with reference to the following embodiments and drawings of the invention.

### Advantageous Effects of Invention

According to the present disclosure, T cells with enhanced glucose uptake ability and/or a related cell population can be provided, and by using such cells and/or the related cell population, chimeric antigen receptor (CAR) T cells and/or a related cell population that function without becoming starved or exhausted even in a tumor environment can be provided.

The cells of the present disclosure can be said to be metabolism-enhanced T cells (survivor T cells), or a related cell population, in which a mechanism by which tumor cells survive in a tumor environment is applied to T cells. According to the cells and related cell population of the present disclosure, effective cytotoxic activity and cytokine production ability under a low-glucose environment such as a tumor microenvironment, which could not be achieved by conventional immune checkpoint inhibitor therapy, can be maintained, and a high antitumor effect in vivo can be exerted. In addition, by using a transient glucose transporter expression system in which a glucose transporter is expressed in accordance with an increase in glucose demand, over-differentiation and exhaustion of T cells due to excessive activation can be avoided, and memory phenotype T cells capable of maintaining an effect for a long period can be induced.

### Brief Description of Drawings

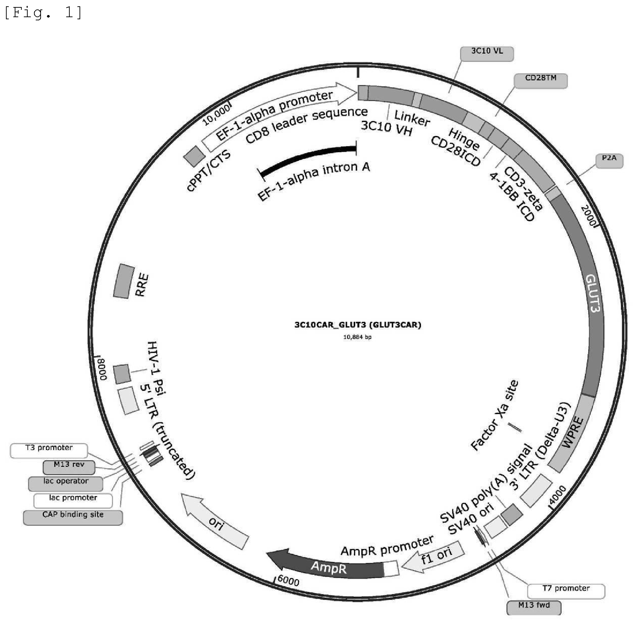

[FIG. 1] FIG. 1 shows an example structure of a metabolically modified anti-EGFRvIII CAR (3C10CAR_GLUT3) lentiviral vector plasmid. A single-chain antibody (scFv) derived from a mouse-derived antibody (clone 3C10) targeting EGFRvIII expressed in glioblastoma (GBM) and the like was constructed, and the single-chain antibody was linked to CD8 hinge, a CD28 transmembrane portion (CD28TM), a CD28 intracellular domain (CD28ICD), 4-1BBICD, and CD3z to construct a CAR. A high-affinity glucose transporter (GLUT3) was encoded downstream of the CAR via a P2A sequence. The drawing shows an example in which the 3C10CAR_GLUT3 was incorporated into a third-generation lentiviral vector plasmid having an EF-1a promoter.

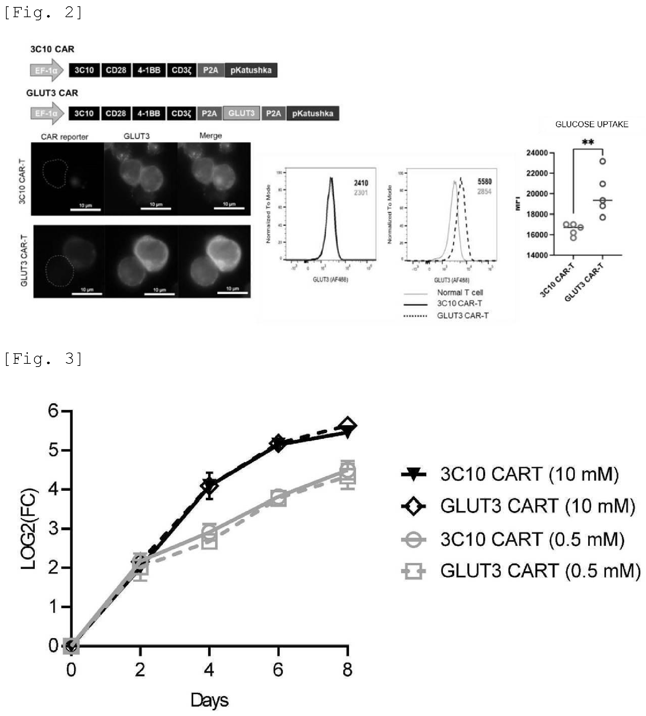

[FIG. 2] FIG. 2 shows T-cell surface GLUT3 expression and enhanced glucose uptake. The upper panel shows structures of a conventional CAR (3C10 CAR) and a metabolically modified CAR (GLUT3 CAR). The lower left shows fluorescence microscopy images of CAR and GLUT3 expression on T cells for 3C10 CAR and GLUT3 CAR. The lower center shows GLUT3 expression quantified by fluorescence intensity. The lower right shows quantification results of the amount of glucose uptake into cells by the 2-NBDG method. A conventional CAR (3C10 CAR) and a metabolically modified CAR (GLUT3 CAR) were introduced into healthy donor-derived T cells by gene transfer to obtain CAR-T cells. Uniform GLUT3 expression was observed on the membrane of GLUT3 CAR-T cells by fluorescence microscopy. In addition, in the GLUT3 CAR-T cells, glucose uptake was enhanced compared with T cells and conventional CAR-T cells (3C10 CAR-T cells).
[FIG. 3] FIG. 3 shows results indicating T-cell expansion efficiency equivalent to that of normal CAR-T cells. The X-axis shows the number of days after introduction, and the Y-axis shows LOG2(FC). Peripheral blood mononuclear cells (PBMCs) containing T cells were stimulated with anti-CD3/28 beads, and then CAR was genetically introduced to analyze T-cell proliferation efficiency. Under both normal glucose conditions (10 mM) and low glucose conditions (0.5 mM), GLUT3 CAR-T cells had establishment efficiency equivalent to that of conventional CAR-T cells (3C10 CAR-T cells).

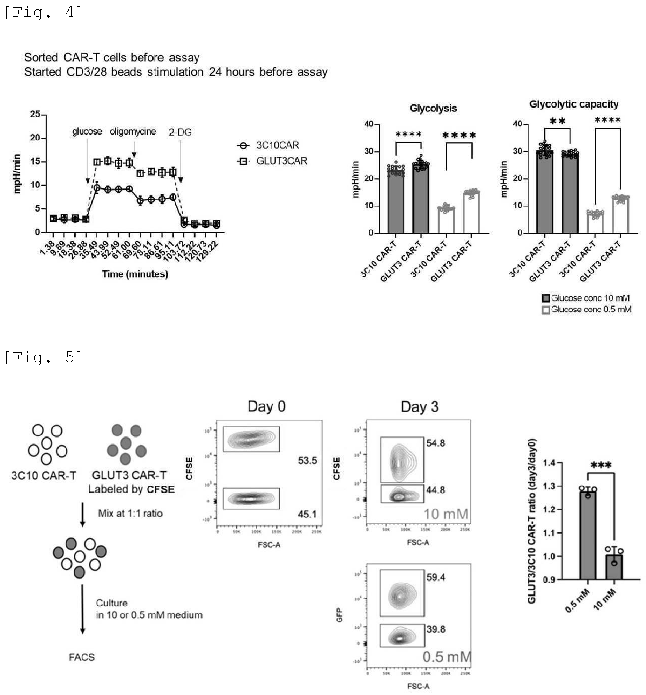

[FIG. 4] FIG. 4 shows results indicating enhancement of T-cell glycolytic ability by GLUT3 expression. The graph on the left shows the glycolytic ability of 3C10 CAR-T cells and GLUT3 CAR-T cells under normal glucose conditions (Glu conc 10 mM) and low glucose conditions (Glu conc 0.5 mM). The lower graph shows the glycolytic ability of 3C10 CAR-T cells and GLUT3 CAR-T cells under normal glucose conditions (Glu conc 10 mM). The left side of the right panel shows values of glycolysis observed after addition of glucose, and the right side shows values of glycolytic capacity observed after addition of Oligomycine. The metabolism of GLUT3 CAR-T cells and 3C10 CAR-T cells was analyzed using a Fluxanalyzer. In both a low-glucose environment and a normal-glucose environment, GLUT3 CAR had enhanced glycolytic ability compared with 3C10 CAR.
[FIG. 5] FIG. 5 shows results indicating that GLUT3 CAR-T cells have a competitive advantage under low glucose conditions. The left panel shows the experimental procedure. The middle panel shows the cell number proportions of 3C10 CAR-T cells and GLUT3 CAR-T cells on day 0 and day 3. The right panel shows the ratio of 3C10 CAR-T cells to GLUT3 CAR-T cells on day 3. 3C10 CAR-T cells (unlabeled) and GLUT3 CAR-T cells (CSFE-labeled) were co-cultured under normal glucose conditions and low glucose conditions. Under normal glucose conditions, survival of both cells was observed to a similar extent, whereas under low glucose conditions, significant survival of GLUT3 CAR-T cells was observed.

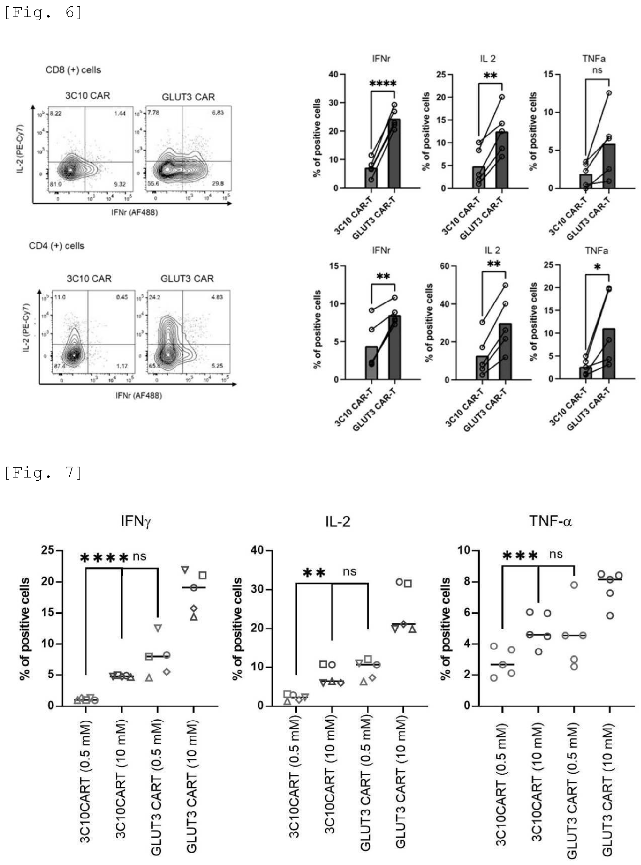

[FIG. 6] FIG. 6 shows results indicating enhanced cytokine production ability of GLUT3 CAR-T cells. The left panel shows intracellular IFNg and IL-2 analysis for CD8-positive cells at the top, and intracellular IFNg and IL-2 amounts for CD4-positive cells at the bottom. The right panel shows the proportions of positive cells for IFNγ, IL-2, and TNF-α. After antigen stimulation of 3C10 CAR-T cells and GLUT3 CAR-T cells, the proportion of cytokine-producing cells was analyzed by FCM. A significant increase in expression of cytokines (IFN-g, IL-2, TNF-a), which are important for T cell maintenance and activation and exertion of antitumor activity, was observed.
[FIG. 7] FIG. 7 shows results indicating the function of GLUT3 CAR-T cells that is maintained even under low glucose conditions. The panels from left to right show the proportions of IFNγ-, IL-2-, and TNF-α-producing cells, respectively, under normal glucose conditions (10 mM) and low glucose conditions (0.5 mM). Cytokine production ability under normal glucose conditions and low glucose conditions was compared. In conventional CAR-T cells (3C10 CAR-T cells), an extreme decrease in function under low glucose conditions was observed. On the other hand, in GLUT3 CAR-T cells, high cytokine production ability was observed under normal glucose conditions, and even under low glucose conditions, GLUT3 CAR-T cells exerted a function comparable to the cytokine production ability of 3C10 CAR-T cells under normal glucose conditions.

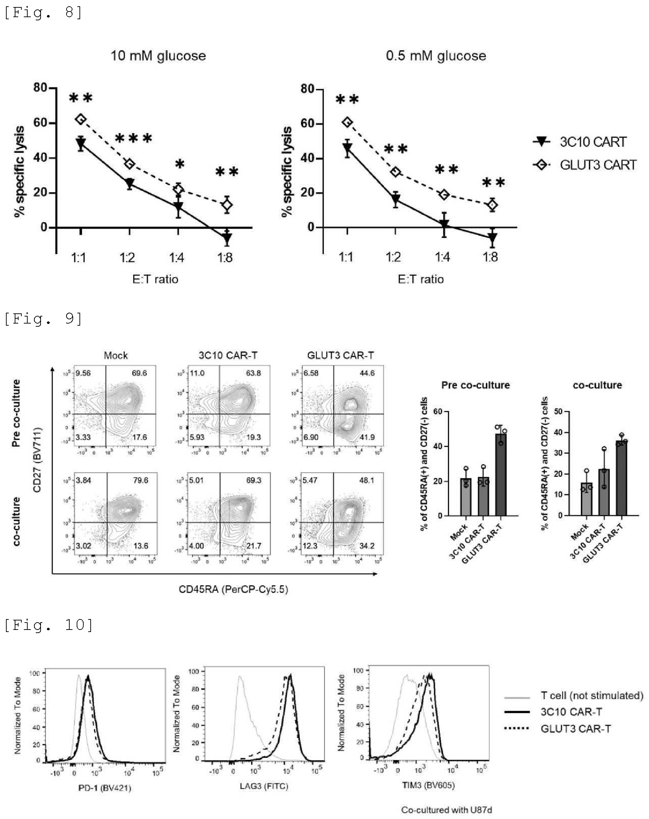

[FIG. 8] FIG. 8 shows results indicating enhanced cytotoxic activity of GLUT3 CAR-T cells. The left panel shows results of cytotoxic activity of 3C10 CAR-T cells and GLUT3 CAR-T cells against the U87d cell line at a glucose concentration of 10 mM, and the right panel shows results of cytotoxic activity of 3C10 CAR-T cells and GLUT3 CAR-T cells against the U87d cell line at a glucose concentration of 0.5 mM. Cytotoxic activity was analyzed using an EGFRvIII-expressing cell line as a target. Cytotoxic activity was analyzed under low glucose conditions and normal glucose conditions, and GLUT3 CAR-T cells showed higher cytotoxic activity than 3C10 CAR-T cells under both environments.
[FIG. 9] FIG. 9 shows results indicating properties imparted by GLUT3 CAR tothe T-cell memory phenotype. The left panel shows expression of CD45 and CD27 before co-culture with the U87d tumor cell line (Preco-culture) and after co-culture (co-culture), and the right panel shows, from the left, the proportions of the CD45RA-positive/CD27-negative fraction before co-culture and after co-culture. Memory phenotypes of 3C10 CAR-T cells and GLUT3 CAR-T cells were compared. In GLUT3 CAR-T cells, a stronger tendency toward differentiation into effector T cells was observed compared with 3C10 CAR.
[FIG. 10] FIG. 10 shows suppression of expression of inhibitory molecules in GLUT3 CAR-T cells. Data for PD-1, LAG3, and TIM3 are shown from the left. Expression of inhibitory molecules under stimulation with EGFRvIII antigen-positive cells was compared. In GLUT3 CAR-T cells, suppression of expression of PD-1, LAG3, and TIM3 was observed.

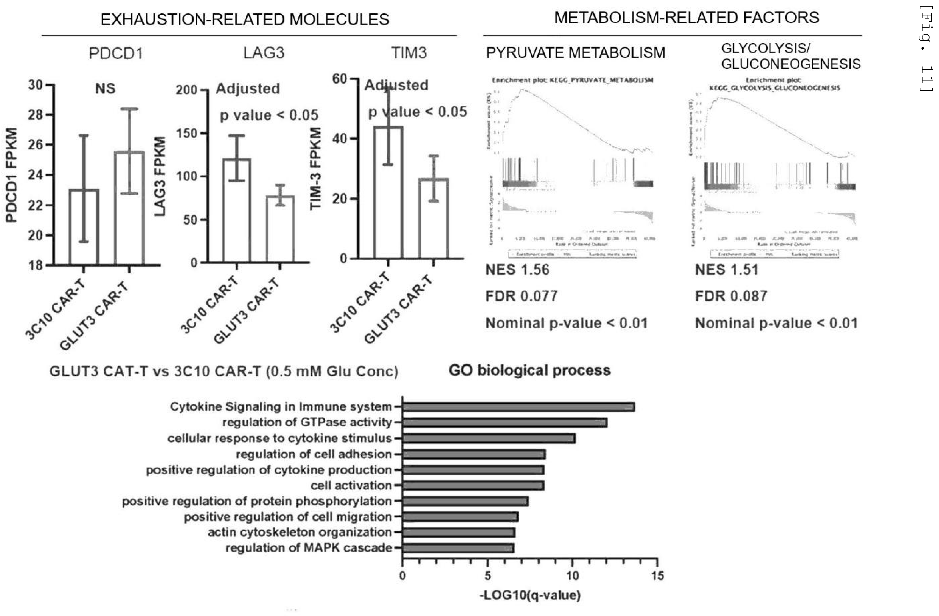

[FIG. 11] FIG. 11 shows mRNA analysis of GLUT3 CAR-T cells (evaluation of metabolism, exhaustion, activation, and differentiation). The upper left panel shows, from the left, PDCD1 expression, LAG3 expression, and TIM3 expression, and the upper right panel shows, from the left, an enrichment plot related to lactic acid metabolism and an enrichment plot related to glucose metabolism. The lower panel shows factors with increased expression based on Gene Ontology. Details of 3C10 CAR-T and GLUT3 CAR-T cells were analyzed based on mRNA expression. In GLUT3 CAR-T cells, as with FCM, a decrease in exhaustion-related molecules and increases in lactate metabolism- and glycolysis-related factors were observed. These factors are factors associated with enhanced T-cell effector functions such as activation and cytokine production.

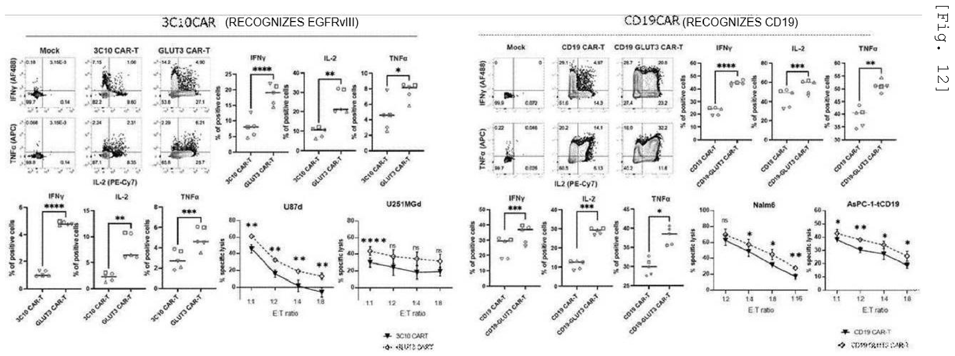

[FIG. 12] FIG. 12 shows demonstration of universality, that is, that similar effects are obtained not only with 3C10 CAR, but also with CD19 CAR. The left panel shows an experiment using 3C10 CAR, and the right panel shows an experiment using CD19 CAR. In each panel, the upper left shows intracellular IL-2, IFNg, and TNF-a analysis of Mock-introduced T cells, 3C10 CAR-T cells, and GLUT3 CAR-T cells, the upper right shows, from the left, the proportions of IFNg-, IL-2-, and TNFa-positive cells among 3C10 CAR-T cells and GLUT3 CAR-T cells under normal glucose conditions (10 mM), and the lower left shows, from the left, the proportions of IFNg-, IL-2-, and TNFa-positive cells among 3C10 CAR-T cells and GLUT3 CAR-T cells under low glucose conditions (0.5 mM). The lower right shows, from the left, cytotoxic activity of 3C10 CAR-T cells and GLUT3 CAR-T cells against the U87d cell line and the U251MGd cell line under low glucose conditions (0.5 mM). It was confirmed that effects similar to those of 3C10 CAR were also obtained when GLUT3 was incorporated into CD19 CAR. It was also shown to be effective against pancreatic cancer cells obtained by causing CD19 to be expressed in a pancreatic cancer cell line.

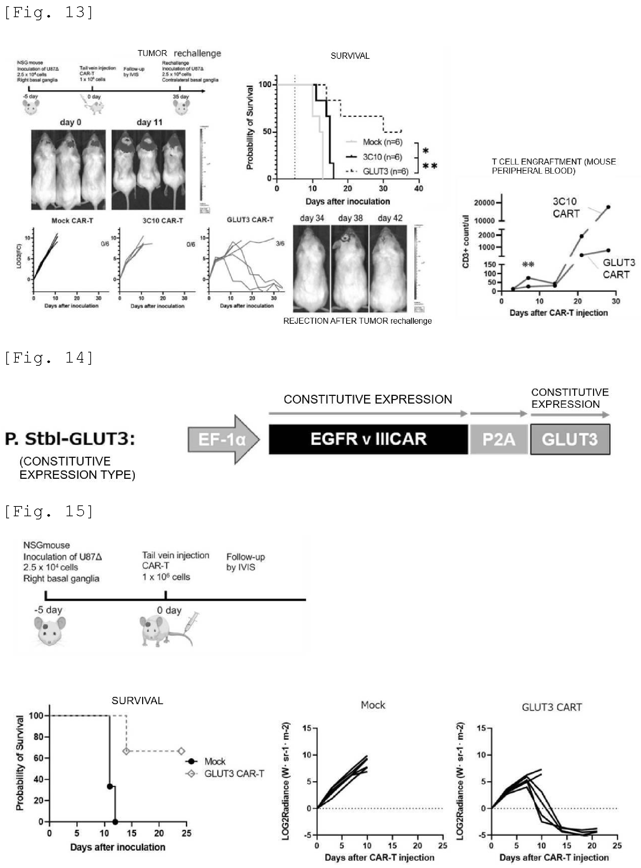

[FIG. 13] FIG. 13 shows that rapid tumor eradication is achieved in an intracranial xenograft model. The upper left panel shows a brief scheme of the experiment. Briefly, this is a model in which 2.5 x 10⁴ cells of the U87d tumor cell line are transplanted into the cranium of NSG mice, which are immunodeficient mice, 1 x 10⁶ CART cells are administered via the tail vein 5 days later, and tumor burden is quantified over time by bioluminescence imaging (BLI). The middle left shows tumor images on day 0 and day 11. The graph at the lower left shows luminescence intensity over time (reflecting tumor burden). The middle-center panel shows Kaplan-Meier curves of each treatment group. Below the middle panel, tumor images on day 34, day 38, and day 42 in tumor rechallenge mice are shown. The right column shows peripheral blood CD3⁺ T-cell counts over time. Antitumor activity was observed in a U87Δ intracranial xenograft model. In the GLUT3 CAR-T cell treatment group, more rapid tumor eradication, improved survival, and superior T-cell expansion were observed compared with the 3C10 CAR-T cell treatment group. In addition, tumor rejection was confirmed by tumor rechallenge in mice cured in the GLUT3 CAR-T cell group.
[FIG. 14] FIG. 14 shows a design of a GLUT3 expression system. The symbols in the schematic diagram respectively indicate the EF-1 promoter (EF-1a), anti-EGFRvIII CAR (EGFRvIII CAR), P2A sequence (P2A), and high-affinity glucose transporter (GLUT3). Stbl-GLUT3 is the same as GLUT3 CAR.
[FIG. 15] FIG. 15 shows evaluation of the antitumor effects of Mock T cells and GLUT3 CAR-T cells in a mouse model. An experimental schematic diagram is shown at the top. A Kaplan-Meier curve after treatment is shown at the upper left. The lower panel shows changes over time in tumor burden quantified by bioluminescence imaging (BLI). In Mock T cells, the tumor was not suppressed, whereas complete remission (CR) was obtained in four mice with GLUT3 CAR-T.

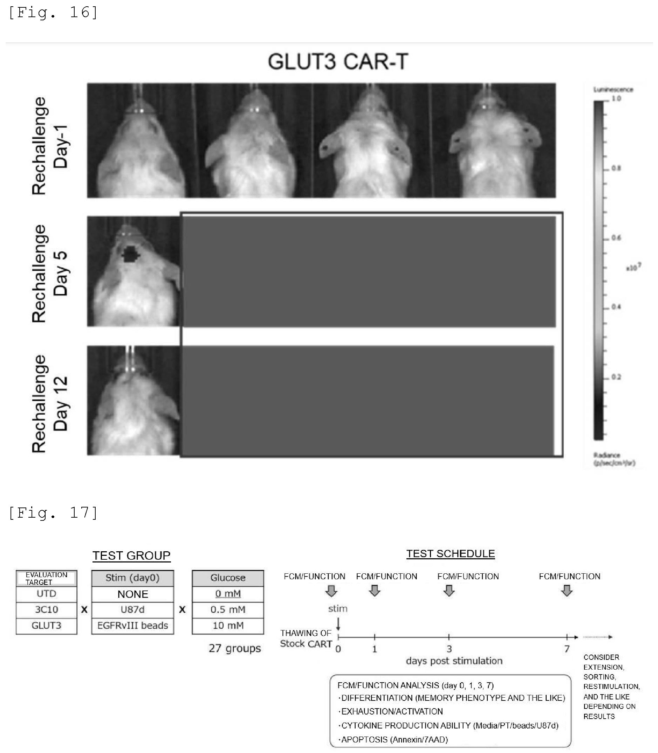

[FIG. 16] FIG. 16 shows a tumor rechallenge experiment (sudden death of mice in the GLUT3 CAR-T cell group). Tumor burden and survival after GLUT3 CAR-T cell treatment are shown (tumor images on day 1 after challenge, day 5 after rechallenge, and day 12 after rechallenge are shown). Tumor (U87d) was re-transplanted into cured mice from the experiment in the previous figure, and rejection ability was evaluated ("surrogate for memory formation"). 1 x 10⁵ tumor cells were re-transplanted, and one of four mice in the GLUT3 CAR-T group achieved CR. However, three out of four mice died (death unrelated to tumor growth).
[FIG. 17] FIG. 17 shows enhancement of long-term survival after stimulation, with each CAR-T cell observed over time under the above conditions. This drawing shows an in vitro (CAR) T-cell stimulation and culture test plan. Untransduced T cells (UTD) , 3C10 CAR-T cells, and GLUT3 CAR-T cells are used as evaluation targets, and an unstimulated group and stimulated groups (stimulation with the U87d cell line or stimulation with EGFRvIII magnetic beads) are provided. Furthermore, each group is cultured under glucose-free conditions (0 mM), low glucose conditions (0.5 mM), and normal glucose conditions (10 mM) (27 groups in total). For each group, the analyses shown in the schedule on the right are performed over time. Under low-glucose conditions, conventional CAR-T 3C10 does not exhibit effective cytokine production, whereas GLUT3 CAR-T exhibits effective cytokine production ability. However, due to excessive glucose uptake, GLUT3 CAR-T undergoes terminal differentiation over time (day 3 or day 7) into CCR7-negative effector memory T cells (Tem) or terminally differentiated RA-positive T cells (Temra), loses stemness, and undergoes apoptosis. When CAR-T cells (established under normal glucose conditions) are suddenly (day 0) placed under low-glucose conditions of 0 mM or 0.5 mM, highly glucose-dependent GLUT3 CAR-T is prone to apoptosis.

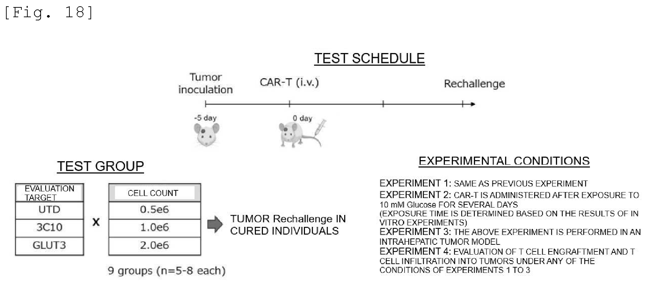

[FIG. 18] FIG. 18 shows enhancement of long-term survival in vivo. The above antitumor effect, survival, and adverse events are examined in a system similar to that of the mice described above. In this drawing, the U87d tumor cell line is transplanted intracranially into NSG mice, which are immunodeficient mice, and 5 days later, Mock-introduced T cells (UTD), 3C10 CAR-T cells, and GLUT3 CAR-T cells are administered via the tail vein, and quantification of antitumor effects by bioluminescence imaging and survival are analyzed. In Experiment 1, established CAR-T cells are used as they are; in Experiment 2, cells are administered after exposure to a high-glucose environment; in Experiment 3, a tumor is transplanted into the liver, in which a low-glucose, high-lactic-acid environment has been reported, and the experiment is performed; and in Experiment 4, T cell engraftment and infiltration into tumors are evaluated. In Experiment 1, GLUT3 CAR-T cells show superior antitumor activity. In Experiment 2, apoptosis and over-differentiation occur due to GLUT3 CAR-T overactivation caused by glucose exposure, and antitumor activity is attenuated. In rechallenge, tumor rejection is achieved with GLUT3 CAR-T cells. In Experiment 4, superior CAR-T cell engraftment and T-cell infiltration into tumors are observed in GLUT3 CAR-T cells. Experiment 3 is an exploratory experiment in a liver tumor model in which immunosuppression due to a low-glucose, high-lactic-acid environment has been suggested, and similar results to those in Experiment 2 are expected.

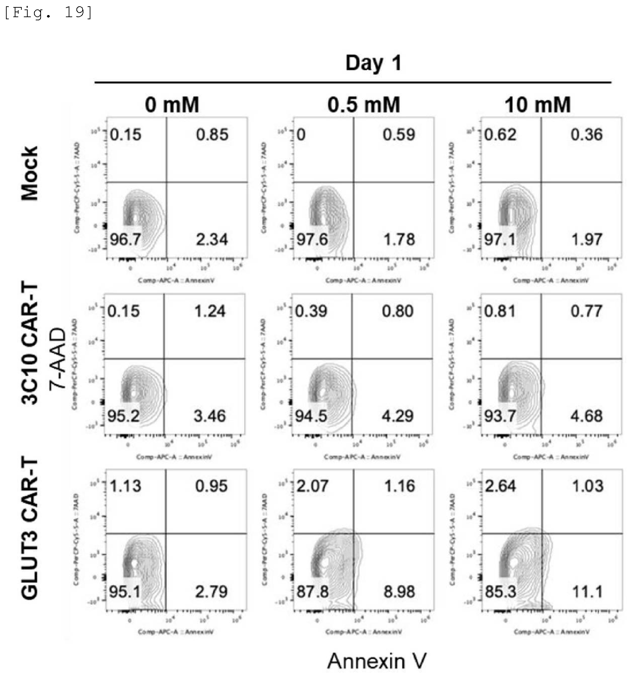

[FIG. 19] FIG. 19 shows data indicating enhancement of long-term survival after stimulation (in vitro data). From the left, the results of cell death in GLUT3 CAR-T cells under 0 mM, 0.5 mM, and 10 mM conditions are shown. Cell death in each CAR-T cell was examined using Annexin V and 7-AAD. In GLUT3 CAR-T cells, in an unstimulated state, the rates of apoptosis and cell death under no- and low-glucose conditions (0 mM and 0.5 mM) were comparable to those under normal-glucose conditions (10 mM).

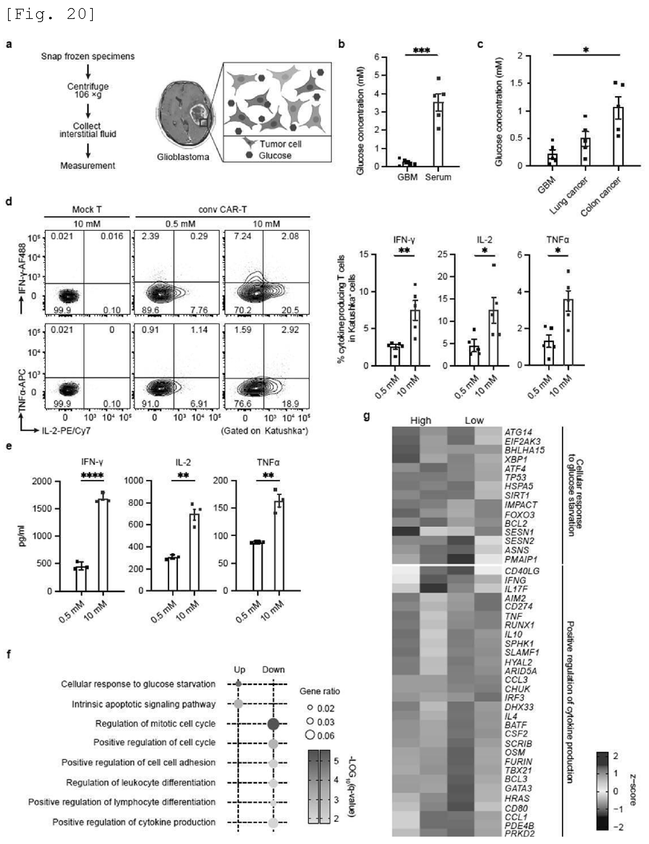

[FIG. 20] FIG. 20 is a drawing showing that a low-glucose state impairs the function of CAR-T cells. (a-c) Interstitial fluid was collected by a low-speed centrifugation method from surgical specimens or biopsy specimens of patients (n=5 each) with glioblastoma (GBM), non-small cell lung cancer (Lung cancer), or colorectal cancer (Colon cancer), and glucose concentrations were examined. (a) Experimental schema (b) Glucose concentrations in the interstitial fluid of GBM specimens and in the paired serum specimens Data are shown as mean ±SEM. Statistical analysis by Student's t-test; ***, P<0.001 (c) Glucose concentrations in interstitial fluid of GBM, non-small cell lung cancer (lung cancer), and colorectal cancer (colorectal cancer) specimens Data are shown as mean ±SEM. Statistical analysis by one-way analysis of variance; *, P<0.05 (d,e) Mock-introduced T cells (Mock T cells) and conventional EGFRvIII CAR-T cells were prepared from PBMCs of healthy subjects (n=5) (d) Cytokine production by Mock-transduced T cells (Mock T) and conv EGFRvIII CAR-T cells (conv CAR-T) when stimulated with EGFRvIII-expressing tumor cells (U-87 MGΔ) under low-glucose (0.5 mM) or high-glucose (10 mM) conditions was examined by intracellular cytokine staining. Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; **, P<0.01 (e) Cytokine (IFN-γ, IL-2, TNFα) production by conv EGFRvIII CAR-T cells co-cultured with U-87 MGΔ cells for 16 hours under low-glucose (0.5 mM) or high-glucose (10 mM) conditions was measured by ELISA. Data are shown as mean ±SEM. Statistical analysis by Student's t-test; **, P<0.01; ****, P<0.0001 (f) Gene expression of EGFRvIII CAR-T cells 24 hours after EGFRvIII bead stimulation under low-glucose (0.5 mM) or high-glucose (10 mM) conditions. Pathways enriched under low-glucose conditions compared with high-glucose conditions are shown according to the Molecular Signatures Database. (g) Gene expression of selected genes is shown in a heat map. Gene expression was normalized by row. All experiments were performed at least twice.

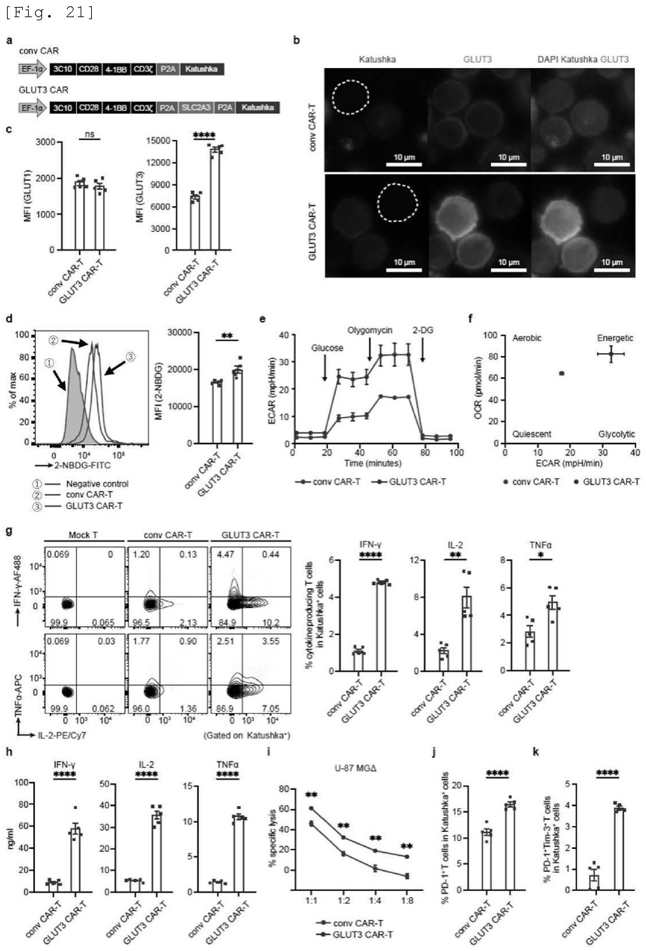

[FIG. 21] FIG. 21 is a drawing showing that overexpression of GLUT3 enhances metabolic fitness of CAR-T cells and increases cytotoxicity and cytokine production under low-glucose conditions. (a) Constructs of a conventional EGFRvIII CAR (conv CAR, upper) and an EGFRvIII CAR stably expressing GLUT3 (GLUT3 CAR, lower) (b) Immunofluorescence staining of conv EGFRvIII CAR-T cells (conv CAR-T) and GLUT3 EGFRvIII CAR-T cells (GLUT3 CAR-T) White dotted circles indicate T cells that were not transduced with CAR. Katushka (red), 4,6-diamino-2-phenylindole (DAPI: blue), GLUT3 (green) (c) Expression levels of GLUT1 (left) and GLUT3 (right) in CAR-T cells A summary of mean fluorescence intensity (MFI) is shown. Data are shown as mean ±SEM. Statistical analysis by Student's t-test; ns, not significant; ****, P<0.0001 (d) Glucose uptake by CAR-T cells as measured by a glucose analog 2-NBDG uptake assay Representative histograms (left) and a summary of mean fluorescence intensity (MFI) (right) are shown. Data are shown as mean ±SEM. Statistical analysis by Student's t-test; **, P<0.01 (e,f) Glycolytic ability (e) and metabolic phenotype (f) of CAR-T cells under low-glucose (0.5 mM) conditions as analyzed by a flux analyzer Data are shown as mean ±SD. (g,h) CAR-T cells were prepared from PBMCs of healthy subjects (n=5) (g) Cytokine production upon stimulation with EGFRvIII-expressing tumor cells (U-87 MGΔ) under low-glucose (0.5 mM) conditions was examined by intracellular cytokine staining. Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; **, P<0.01; ****, P<0.0001 (h) Cytokine (IFN-γ, IL-2, TNFα) production by CAR-T cells co-cultured with U-87 MGΔ cells for 16 hours under low-glucose (0.5 mM) conditions was measured by ELISA. Data are shown as mean ±SEM. Statistical analysis by Student's t-test; ****, P<0.0001 (i) Cytotoxicity of CAR-T cells under low-glucose (0.5 mM) conditions was measured by a luciferase-based assay. CAR-T cells and luciferase-expressing target cells (U-87 MGΔ-Luc) were co-cultured at the indicated effector-to-target (E : T) ratios for 24 hours, and specific lysis was calculated based on luciferase activity. Data are shown as mean ±SEM. Statistical analysis by Student's t-test; **, P<0.01 (j,k) Expression of exhaustion markers (PD-1 and Tim-3) by CAR-T cells A summary of the frequencies of PD-1⁺ CAR-T cell (j) and PD-1⁺Tim-3⁺ CAR-T cell (k) populations is shown. Data are shown as mean ±SEM. Statistical analysis by Student's t-test; ****, P<0.0001

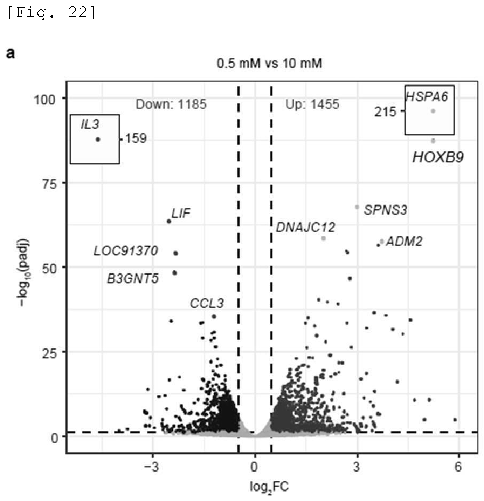

[FIG. 22] FIG. 22 is a drawing showing that a low-glucose state impairs the function of CAR-T cells. (a) Volcano plot showing genes differentially expressed in conv EGFRvIII CAR-T cells cultured under low-glucose (0.5 mM) conditions compared with high-glucose (10 mM) conditions The X-axis represents log₂ (fold change) (log₂ FC), and the Y-axis represents -log₁₀ (adjusted p value) [(-log₁₀(padj))]. The dotted lines represent the cutoff value for log₂ (fold change), x=±1.4, and the cutoff value for -log₁₀ (adjusted p value), y=0.1. The top 5 genes and bottom 5 genes are highlighted in green and purple, respectively.

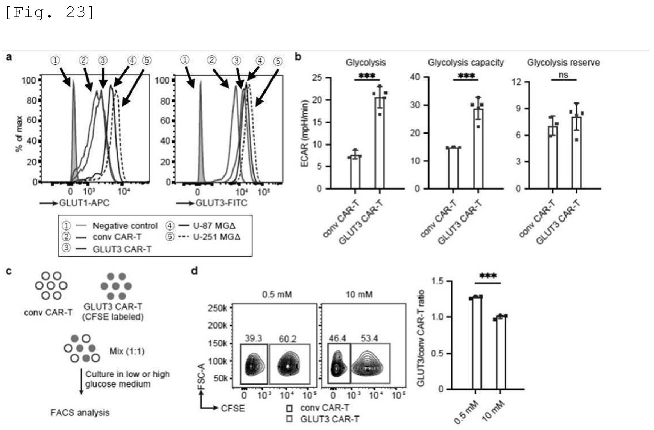

[FIG. 23] FIG. 23 is a drawing showing that overexpression of GLUT3 enhances metabolic fitness of CAR-T cells. (a) Expression of GLUT1 (left) and GLUT3 (right) in conv EGFRvIII CAR-T cells (conv CAR-T), GLUT3 EGFRvIII CAR-T cells (GLUT3 CAR-T), U-87 MGΔ cells, and U-251 MGΔ cells Representative histograms are shown. (b) Glycolytic parameters of CAR-T cells cultured under low-glucose (0.5 mM) conditions as measured by a glycolysis stress test Data are shown as mean ±SD. Statistical analysis by Student's t-test; ns, not significant; ***, P<0.001 (c,d) Competition assay (72-hour culture) of CAR-T cells under low-glucose (0.5 mM) or high-glucose (10 mM) conditions (c) Experimental schema (n=3) (d) Representative contour plots (left) and a summary of the proportions of each CAR-T-cell population (right) Data are shown as mean ±SEM. Statistical analysis by Student's t-test; ***, P<0.001

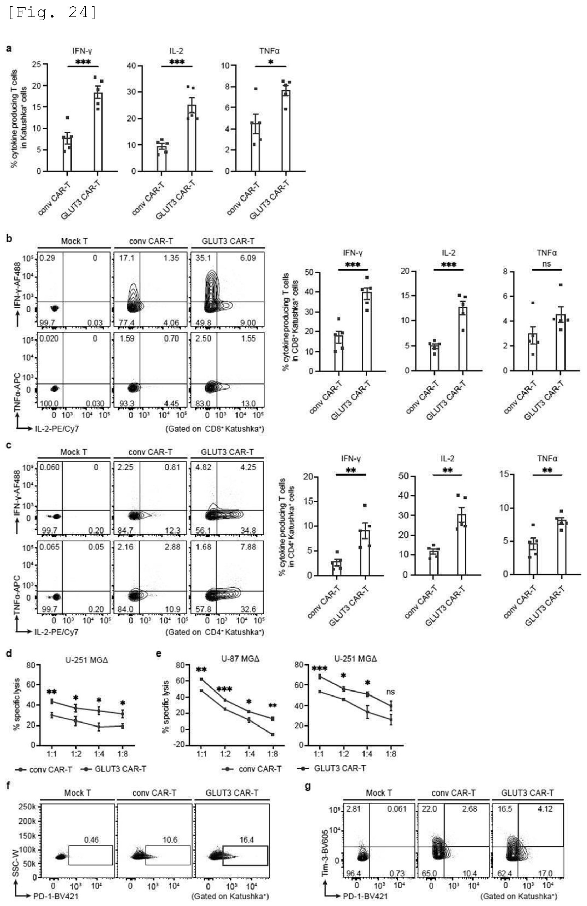

[FIG. 24] FIG. 24 is a drawing showing that stable expression of GLUT3 improves the function of CAR-T cells under low-glucose conditions. (a-c) Cytokine production by mock T cells (Mock T), conv EGFRvIII CAR-T cells (conv CAR-T), and GLUT3 EGFRvIII CAR-T cells (GLUT3 CAR-T) (n=5) when stimulated with EGFRvIII-expressing tumor cells (U-87 MGΔ) under high-glucose (10 mM) conditions was examined by intracellular staining. (a) Summary of the frequencies of cytokine (IFN-γ, IL-2, TNFα)-producing CD3⁺ CAR-T cells Data are shown as mean ±SEM. Statistical analysis by Student's t-test; *, P<0.05; ***, P<0.001 (b) Representative contour plots (left) and a summary of the frequencies of cytokine (IFN-γ, IL-2, TNFα)-producing CD8⁺ CAR-T cells (right) Data are shown as mean ±SEM. Statistical analysis by Student's t-test; ns, not significant; ***, P<0.001 (c) Representative contour plots (left) and a summary of the frequencies of cytokine (IFN-γ, IL-2, TNFα)-producing CD4⁺ CAR-T cells (right) Data are shown as mean ±SEM. Statistical analysis by Student's t-test; **, P<0.01 (d,e) Cytotoxicity of CAR-T cells against U-87 MGΔ cells and U-251 MGΔ cells under low-glucose (0.5 mM) or high-glucose (10 mM) conditions was determined by a luciferase-based assay. CAR-T cells and luciferase-expressing target cells (U-87 MGΔ-Luc and U-251 MGΔ-Luc) were co-cultured at the indicated effector-to-target (E : T) ratios for 24 hours, and specific lysis was calculated based on luciferase activity. (d) Cytotoxicity of CAR-T cells against U-251 MGΔ-Luc cells under low-glucose (0.5 mM) conditions Data are shown as mean ±SEM. Statistical analysis by Student's t-test; *, P<0.05; **, P<0.01 (e) Cytotoxicity of CAR-T cells against U-87 MGΔ-Luc cells or U-251 MGΔ-Luc cells under high-glucose (10 mM) conditions Data are shown as mean ±SEM. Statistical analysis by Student's t-test; ns, not significant; *, P<0.05; **, P<0.01; ***, P<0.001
   (F,G) Expression of exhaustion markers (PD-1, f; PD-1 and Tim-3, g) by the indicated CAR-T cell populations Representative contour plots are shown.

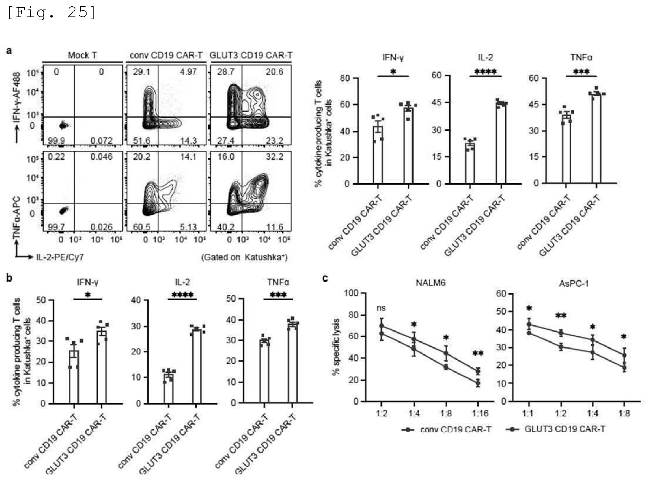

[FIG. 25] FIG. 25 is a drawing showing that improvement in CAR-T cell function by stable expression of GLUT3 does not depend on the type of scFv. (a,b) Mock T cells (Mock T), conv CD19 CAR-T cells (conv CD19 CAR-T), and GLUT3 CD19 CAR-T cells (GLUT3 CD19 CAR-T) were prepared from PBMCs of healthy subjects (n=5). (a) Cytokine production by the indicated CAR-T cells upon stimulation with CD19-expressing tumor cells (NALM6) under low-glucose (0.5 mM) conditions, as determined by intracellular cytokine staining Representative contour plots (left) and a summary of frequencies of cytokine (IFN-γ, IL-2, TNFα)-producing cells (right) are shown. Data are shown as mean ±SEM. Statistical analysis by Student's t-test; *, P<0.05; ***, P<0.001; ****, P<0.0001
   (b) Cytokine (IFN-γ, IL-2, TNFα) production by conv CD19 CAR-T cells and GLUT3 CD19 CAR-T cells co-cultured with NALM6 for 16 hours under low-glucose (0.5 mM) conditions was measured by ELISA. Data are shown as mean ±SEM. Statistical analysis by Student's t-test; *, P<0.05; ***, P<0.001; ****, P<0.0001
   (c) Cytotoxicity of conv CD19 CAR-T cells and GLUT3 CD19 CAR-T cells against NALM6 and CD19-expressing AsPC-1 cells under low-glucose (0.5 mM) conditions was determined by a luciferase-based assay. CAR-T cells and luciferase-expressing target cells (NALM6-Luc and CD19-expressing AsPC-1-Luc) were co-cultured at the indicated effector-to-target (E : T) ratios for 24 hours, and the specific lysis rate was evaluated based on luciferase activity. Data are shown as mean ±SEM. Statistical analysis by Student's t-test; ns, not significant; *, P<0.05; **, P<0.01

### Description of Embodiments

Hereinafter, the present disclosure will be described by way of the best mode. Throughout the present specification, the expression of singular forms is to be understood as including the concept of plural forms unless otherwise stated. Therefore, it should be understood that singular articles (for example, "a", "an", "the", and the like in English) also include the concept of their plural forms unless specifically stated otherwise. In addition, the terms used in the present specification are to be understood as being used in the sense commonly used in the art unless otherwise stated. Therefore, unless otherwise defined, all technical and scientific terms used in the present specification have the same meanings as generally understood by those skilled in the art to which the present disclosure belongs. In the case of conflict, the present specification (including definitions) will control.

Hereinafter, definitions and/or basic technical contents of terms particularly used in the present specification will be described as appropriate.

In the present specification, "about" means ±10% of the numerical value that follows. For example, "about 20" is intended to include "18 to 22". A numerical range includes all numerical values between both endpoints and the numerical values of both endpoints. "About" regarding a range applies to both endpoints of the range. Therefore, for example, "about 20 to 30" is intended to include "18 to 33".

In the present specification, the term "T cell" is used in a broad sense used in the art, and refers to lymphocytes generated in bone marrow that migrate to the thymus and mature. T cells may be CD45-positive and CD3-positive cells among normal fractions of peripheral blood and bone marrow-derived mononuclear cells. As the T cells to be used, T cells isolated from a donor, particularly a human donor, may be used, but are not limited thereto. Examples of T cells and cells derived therefrom include isolated T cells that have not been passaged in culture, T cells that have been passaged and maintained under cell culture conditions without being immortalized, and T cells that have been immortalized and can be maintained indefinitely under cell culture conditions. It is known that multiple types of T cells exist depending on their functions, and examples thereof include effector memory T cells (T_{eff}), naive T cells (Tnv), stem cell memory T cells (Tscm), central memory T cells (Tcm), terminally differentiated RA-positive T cells (Temra), and the like. In the present specification, the term "peripheral T cell" refers to a T cell present outside the thymus, and can be obtained from peripheral blood, lymph nodes, or other tissues. In the present specification, when referring to "peripheral T cells", it is sufficient that peripheral T cells are contained in a cell group, and the T cells do not need to be isolated. A cell fraction containing various lymphocytes in addition to T cells, such as peripheral blood mononuclear cells (PBMCs), may be used.

In the present specification, the term "cell population" refers to a population containing two or more cells, and may be, for example, a state in which cells are gathered together in a planar manner or a cell mass formed by adhesion of cells to each other three-dimensionally. In addition, the "cell population" may be formed of a single type of cell or may contain a plurality of types of cells. When referring to a cell population of T cells of the present disclosure, it is sufficient that the cell population contains at least one cell, such as Tnv, Tscm, Tcm, or Temra, that can have an effector function at a place where the cell is to function (typically in vivo).

In the present specification, the term "flow cytometry" refers to a technique for measuring the particle number and individual physical, chemical, and biological properties of cells, organisms, and other biological particles suspended in a liquid. An apparatus using this technique is referred to as a "flow cytometer". In the present disclosure, "positive" and "negative" for cell markers (for example, FoxP3, CTLA4, Helios, CD103, and the like) are determined by flow cytometry, as commonly used in the art. More specifically, in flow cytometry, cells are aligned and flowed in a single line, and the number of cells is counted by a spectroscopic technique. For example, the number of target cells is counted by irradiating cells labeled with fluorescence or a luminescent enzyme with laser light, and detecting fluorescence or luminescence signals emitted from the cells by a detector such as a photodiode. In addition, detection results from the detector can be imported into a computer to generate and display a two-dimensional plot. This makes it possible to easily grasp the presence or absence of target cells, the number thereof, and the like.

In the present specification, the term "effector function" refers, in T cells, to biological activities such as cytotoxic activity, cytokine production, and division/proliferation, which are caused by signals via TCR or CAR, and refers, in other immune cells, to biological activities caused by the Fc region of an antibody. Examples of effector functions include C1q binding and complement-dependent cytotoxicity (CDC), Fc receptor binding, antibody-dependent cellular cytotoxicity (ADCC), phagocytosis, downregulation of cell surface receptors (for example, B cell receptors), and activation of B cells. In the present disclosure, whether T cells have an effector function can be determined by confirming that the T cells have cytotoxic activity, cytokine production, and division/proliferation using methods exemplified in Examples and the like.

In the present specification, the term "effector cell" or "effector T cell" means an immune cell that is a T cell having an effector function and that exerts a cytotoxic action on a target cell, and may also be referred to as T_{eff}. In the present specification, whether a cell is an "effector" cell can be determined by determination methods such as a cytotoxicity assay, surface antigen analysis by flow cytometry, and intracellular cytokine staining. Effector T cells are immune cells that execute effector functions, and mediate antibody-dependent cellular cytotoxicity (ADCC) and the like. Effector T cells include, for example, peripheral blood mononuclear cells (PBMCs), natural killer (NK) cells, monocytes, macrophages, cytotoxic T cells, neutrophils, and the like, and can be isolated from natural tissues such as blood.

In the present specification, the term "precursor cells of effector T cells (T_{eff})" refers to cells that are not effector cells, but acquire an effector function by being transferred to a place where they are to function (for example, in vivo) or by other stimulation. Such cells include lymphoid precursor cells, Tnv, Tscm, Tcm, Temra, and the like. Precursor cells of effector T cells are activated at an early stage of an immune response and mature into effector T cells through proliferation and differentiation, whereas some precursor cells have an ability to differentiate into memory T cells. These precursor cells have specific gene expression patterns and metabolic states, which contribute to prolongation of memory function. Several molecular and metabolic properties are involved in this prolongation of memory function. First, precursor cells of effector T cells have specific characteristics in their metabolic programs. In these cells, oxidative phosphorylation, rather than the glycolytic pathway (glycolysis) characteristic of effector T cells, is predominant, and this metabolic pathway has high energy efficiency and enables long-term survival of the cells. This metabolic property is an important factor supporting long-term maintenance of function as memory T cells. Next, the role of transcription factors is also important. In precursor cells of effector T cells, transcription factors such as T-bet and Eomes are expressed, and these promote differentiation into memory T cells while enabling their long-term survival and function. In addition, these precursor cells expressing surface markers such as CD62L and CCR7 have the ability to migrate to secondary lymphoid tissues. Through this homing function, the precursor cells receive survival signals necessary in secondary lymphoid tissues and contribute to maintenance of memory function. Furthermore, suppression of antigen stimulation is also involved in prolongation of memory function. Some precursor cells are released from antigen stimulation, whereby complete differentiation into effector T cells is suppressed, and instead, properties as memory T cells are retained. By this mechanism, an excessive effector response is suppressed, and at the same time, memory function is retained. In addition, the cytokine environment around the cells also plays an important role. In particular, cytokines such as IL-7 and IL-15 provide survival signals necessary for maintenance of memory T cells, and this greatly contributes to prolongation of memory function. Due to these factors, precursor cells of effector T cells survive for a long period of time compared with ordinary effector T cells and retain memory function that enables a rapid and strong secondary immune response. This property is considered to play an important role not only in immune defense against infectious diseases but also in cancer immunotherapy and vaccine development. Thus, understanding the properties of precursor cells of effector T cells has great significance in both basic immunology research and clinical applications.

In precursor cells of effector T cells, the phrase "a memory T cell phenotype is maintained" refers to a phenomenon in which, in the process of differentiation into effector T cells, some precursor cells exist while retaining phenotypes characteristic of memory T cells (for example: surface markers, metabolic states, expression of transcription factors, and the like). Such precursor cells survive even after completion of an immune response and have the ability to provide immune memory over a long period of time. This phenomenon is considered to be an important mechanism for achieving both the process in which T cells exert an effector function and the memory function that enables long-term survival and a rapid secondary immune response. The characteristics of this phenomenon will be described. First, precursor cells of effector T cells maintain surface markers characteristic of memory T cells. These include homing molecules such as CD62L and CCR7, and by expression of these molecules, the precursor cells retain the ability to migrate to secondary lymphoid tissues. Next, these precursor cells are also characterized by maintenance of their metabolic state. Specifically, oxidative phosphorylation is predominant over the glycolytic pathway, and long-term survival is enabled by maintaining a metabolic program with high energy efficiency. This metabolic property is an important factor for maintaining function as memory T cells. In addition, the precursor cells also maintain a characteristic balance in expression of transcription factors. Appropriate expression of transcription factors such as T-bet and Eomes plays a role in making differentiation into effector T cells compatible with maintenance of the memory T cell phenotype. Furthermore, the precursor cells have responsiveness to cytokine signals and can receive survival signals such as IL-7 and IL-15. This responsiveness enables the precursor cells to survive for a long period of time while retaining the properties of memory T cells. Finally, these precursor cells can be maintained in an antigen-independent manner. That is, precursor cells of memory T cells have the ability to retain their phenotype even in the absence of an antigen and maintain a state of readiness for a rapid immune response. Due to the above properties, maintenance of the memory T cell phenotype by precursor cells of effector T cells plays an extremely important role in the flexibility of the immune system and establishment of long-term immune memory. These properties are considered to have great significance in defense against infectious diseases, cancer immunotherapy, and vaccine design.

In the present specification, the term "antigen receptor" refers to any molecule that specifically binds to a target antigen and transmits a signal into a cell expressing the receptor. In the present disclosure, the antigen receptor can be introduced into T cells, and may be natural or an artificial molecule. For example, in addition to a T cell receptor (TCR) and a B cell receptor (BCR), a chimeric antigen receptor (CAR) is also encompassed.

In the present specification, the term "chimeric antigen receptor (CAR)" refers to a modified receptor that can impart antigen specificity to cells (for example, immune cells). CAR is also known as an artificial T cell receptor, a chimeric T cell receptor, or a chimeric immune receptor. CAR is a modified receptor that transfers antigen specificity to immune system cells (for example, T cells such as naive T cells, central memory T cells, effector memory T cells, or combinations thereof, NK cells, macrophages, and the like), and may include, for example, an antigen-specific targeting region, an extracellular domain, a transmembrane domain, a costimulatory domain, and/or an intracellular signaling domain. In addition, CAR may also include a CAR using a plurality of antigen-specific targeting regions (often two types) (bispecific CAR). Preferably, the CAR of the present disclosure includes at least one extracellular domain capable of binding to an antigen, at least one transmembrane domain, and at least one intracellular domain.

In the present specification, the term "T cell receptor (TCR)" refers to a receptor present in T cells. TCR is a heterodimeric receptor molecule consisting of two TCR polypeptide chains, and includes αβ-type TCR expressed by ordinary T cells and γδ-type TCR having special functions. The α- and β-chain TCR molecules form a complex with a plurality of CD3 molecules (CD3ζ chain, CD3ε chain, CD3γ chain, and CD3δ chain), transmit intracellular signals after antigen recognition, and initiate various immune responses. Endogenous antigens, such as viral antigens that have proliferated intracellularly in association with viral infection and cancer antigens derived from cancer cells, are presented as antigen peptides on MHC class I molecules. In addition, antigens derived from foreign microorganisms are taken up by antigen-presenting cells by endocytosis, and after being processed, are presented on MHC class II molecules. These antigens are recognized by TCRs expressed by CD8+ T cells or CD4+ T cells, respectively. It is also known that costimulatory molecules such as CD28, ICOS, and OX40 molecules are important for stimulation mediated by TCR molecules. With respect to αβ-type TCR, the respective gene products of α and β are considered to exhibit specificity through a unique combination.

In the present specification, the term "glucose uptake ability" refers to the ability to take up glucose into cells per unit time. Uptake of glucose into cells can be evaluated by a method such as a 2-NBDG uptake assay.

In the present specification, the term "enhancement of glucose uptake ability" refers to improvement in glucose uptake ability by any method, and improvement in glucose uptake ability can be achieved by modifying immune cells (for example, T cells) so as to express one or more types of glucose transporters and the like, and/or modifying the immune cells so that expression of a glucose transporter is enhanced. Such genetically modified immune cells are expected to show high glucose uptake, for example, in a low-glucose environment (for example, in a tumor microenvironment). Therefore, immune cells co-expressing one or more types of glucose transporters and a chimeric receptor polypeptide may exhibit excellent physiological activity (for example, under a tumor microenvironment such as low-glucose conditions, optionally in the presence of a therapeutic antibody), such as cell proliferation, activation (for example, cytokine production, for example, increased production of IL-2 or IFNγ), cytotoxicity, and/or in vivo antitumor activity.

In the present specification, the term "glucose transporter" (GLUT) refers to a transporter that performs facilitated diffusion-type transport of glucose (dextrose). For GLUT, molecular species of GLUT1 to GLUT14 have been reported. Among them, for example, GLUT1 is expressed in a wide range of tissues and is responsible for constitutive baseline glucose transport, GLUT2 operates when sugar concentration increases and enhances glucose uptake, GLUT3 plays a central role in the central nervous system and exhibits a transport function even under low glucose concentrations, and GLUT4 operates in response to insulin stimulation.

In the present specification, the term "tumor microenvironment" is used interchangeably with "tumor environment", and the tumor microenvironment (TME) refers to a local biological environment surrounding tumor tissue and is a concept including various factors involved in tumor progression, proliferation, metastasis, drug resistance, and the like. This environment includes not only tumor cells but also non-tumor cells such as immune cells (for example, T cells and macrophages), fibroblasts, vascular endothelial cells, and adipocytes. In addition, humoral factors such as extracellular matrix (ECM), cytokines, chemokines, and growth factors are also important components. Furthermore, the tumor microenvironment is also affected by physical factors such as oxygen concentration, pH, and nutritional state. Intercellular interactions and changes in physical factors in the tumor microenvironment are considered to play an important role in tumor malignancy and therapeutic responsiveness. In particular, an immunosuppressive microenvironment has attracted attention as a factor that reduces the effects of immunotherapy. In the present invention, the term "tumor microenvironment" refers to a collection of biological and physical factors including these elements and interactions thereof, and is an important target in tumor therapy and diagnosis.

In the present specification, the term "tumor microenvironment conditions" means a tumor microenvironment or conditions equivalent thereto. Therefore, the "tumor microenvironment conditions" mean a broad concept including, in addition to conditions constituting a tumor microenvironment, similar or equivalent environmental conditions under which a tumor is placed in vivo. Specifically, the tumor microenvironment conditions include not only the local biological environment surrounding tumor tissue but also endogenous and exogenous elements that affect a tumor when the tumor is present in vivo. This environment includes interactions between tumor cells and non-tumor cells (for example, immune cells, fibroblasts, vascular endothelial cells, and the like), humoral factors such as extracellular matrix (ECM), cytokines, chemokines, and growth factors, and physical factors such as oxygen concentration, pH, nutritional state, and physical pressure. In addition, the "tumor microenvironment conditions" also include situations in which a tumor interacts with a broad physiological environment in vivo, and therefore are not limited to the local environment around the tumor. In the present invention, the "tumor microenvironment conditions" comprehensively represent broad environmental conditions encountered by a tumor in vivo, and include all factors that affect tumor progression and therapeutic responsiveness.

In the present specification, the term "modification" broadly refers to any structural, functional, or expression-related change made to a gene (including a nucleic acid and a protein corresponding thereto), and includes naturally occurring or artificial changes. Specifically, the modification includes a sequence change by substitution, deletion, insertion, addition, or a combination thereof of a nucleotide sequence, a change in the amino acid sequence or properties of an encoded protein, or chemical modification for the purpose of adding or improving functionality (for example: glycosylation, phosphorylation, acetylation, and the like). In addition, the "modification" also includes gene introduction from the outside (for example: gene introduction via transfection or a viral vector), transformation, or knockout or knock-in of a specific gene using genome editing technology, suppression of gene expression (for example: suppression by RNA interference or CRISPR technology), and the like. Furthermore, epigenetic modification (for example: DNA methylation, histone modification, and the like) and modification of expression regulatory elements are also included. In the present specification, the term "modification" comprehensively refers to any form of change that affects the structure, expression, function, or biological behavior of a gene, and this includes not only endogenous changes but also changes by external introduction or manipulation.

In the present specification, the "disease" is interpreted in the broadest sense, refers to a state in which discomfort or inconvenience has occurred in the mind or body of a human being or an animal, and refers to any state that cannot be said to be a healthy state, such as a disease, a disorder, and various symptoms, even if not specifically defined. Diseases that can be targeted by the present disclosure include, but are not limited to, diseases to which an immune response may be related, for example, cancer, autoimmune diseases, allergies, and infectious diseases.

In the present specification, the phrase "a subject has an immune response" to a certain component or substance means that some immune reaction occurs against the component or substance. In the subject or a biological component derived from the subject (for example, cells and the like), this can be determined by observing changes in various immune cells or increases and decreases in immune-related substances (for example, cytokines and the like), can be judged by an objective index, and can also be subjectively judged based on the experience of a physician or the like.

In the present specification, whether a subject "has immune memory" with respect to a certain component or substance can be evaluated by measuring, in the subject or a biological component derived from the subject (for example, cells and the like), whether the component or substance (i) antigen-dependently enhances cytokine production in memory CD4-positive T cells or has a proliferation-promoting action, (ii) changes expression of surface antigens of memory-type regulatory T cells, (iii) changes the ratio of Treg to Th1, (iv) induces IFN-γ production from T-bet-positive Th1 cells, (v) changes IFN-γ production ability, (vi) changes IL-2 production ability, (vii) changes TNF-α production ability, or (viii) whether the subject has an antibody specific to the component or substance in blood, and confirming that at least one of these produces a positive result.

In the present specification, the "infectious disease" may be any infectious disease, including any type of infectious disease such as a viral infectious disease (including any viral form such as a single-stranded or double-stranded DNA virus or RNA virus), a bacterial infectious disease, a protozoal infectious disease, or a mycoplasma infectious disease, and may be, for example, tuberculosis, coronavirus, malaria, yellow fever virus, smallpox virus, vaccinia, measles/rubella, polio, epidemic parotitis/MUMPS, rotavirus infection, chicken pox, yellow fever, Ebola, West Nile Fever, Hib infection, pneumococcus infection, whooping cough, Japanese encephalitis, meningococcal infection, salmonella infection, pathogenic E. coli, toxoplasma, zika virus, herpes virus type 1, EBV/Epstein-Barr virus (herpes virus type 4), CMV/cytomegalovirus (herpes virus type 5), influenza, MARS, rabies, and diphtheria.

In the present specification, the term "immune abnormality" refers to any disease, disorder, or condition caused or suspected to be caused at least in part by an abnormality of the immune system. The immune abnormality refers to a state in which an abnormality occurs in immunity due to some cause, and the subject becomes susceptible to infectious diseases or an allergic reaction occurs. Examples of immune abnormalities include, but are not limited to, allergies and autoimmune diseases. When directed against self-antigens, it is generally referred to as an autoimmune disease, and when directed against external antigens, it is referred to as an allergy. When the immune response is strong, it can be said that a state of autoimmune disease occurs against self-antigens and a state of allergy occurs against non-self antigens, whereas when the immune response is weak, it can be said that a state of cancer occurs against self-antigens and an infectious disease occurs against non-self antigens.

In the present specification, the term "autoimmune disease" refers to an immune reaction occurring excessively against a specific self-antigen. An autoimmune disease can be said to be a disease caused by a breakdown of immune tolerance, in which the immune system, which has a role of recognizing and eliminating foreign substances, excessively reacts against and attacks even one's own normal cells and tissues, thereby causing symptoms. Examples of autoimmune diseases or disorders include, but are not limited to, the following: inflammatory responses such as inflammatory skin diseases including psoriasis and dermatitis (for example, atopic dermatitis); systemic scleroderma and sclerosis; reactions associated with inflammatory bowel disease (for example, Crohn's disease and ulcerative colitis); respiratory distress syndrome (including adult respiratory distress syndrome: ARDS); dermatitis; meningitis; encephalitis; uveitis; colitis; glomerulonephritis; allergic conditions such as eczema and asthma, and other conditions involving T cell infiltration and chronic inflammatory responses; atherosclerosis; leukocyte adhesion deficiency; rheumatoid arthritis; systemic lupus erythematosus (SLE) (including, but not limited to, lupus nephritis and cutaneous lupus); diabetes (for example, type I diabetes or insulin-dependent diabetes); multiple sclerosis; Raynaud's syndrome; autoimmune thyroiditis; Hashimoto's thyroiditis; allergic encephalomyelitis; Sjögren's syndrome; juvenile-onset diabetes; immune reactions associated with acute and delayed-type hypersensitivity mediated by cytokines and T lymphocytes typically observed in tuberculosis, sarcoidosis, polymyositis, granulomatosis, and vasculitis; pernicious anemia (Addison's disease); diseases involving leukocyte leakage; central nervous system (CNS) inflammatory disorders; multiple organ injury syndrome; hemolytic anemia (including, but not limited to, cryoglobulinemia or Coombs-positive anemia); myasthenia gravis; antigen-antibody complex-mediated diseases; anti-glomerular basement membrane disease; antiphospholipid syndrome; allergic neuritis; Graves' disease; Lambert-Eaton myasthenic syndrome; bullous pemphigoid; pemphigus; autoimmune polyendocrine disorder; Reiter's disease; stiff-man syndrome; Behçet's disease; giant cell arteritis; immune complex nephritis; IgA nephropathy; IgM polyneuropathy; immune thrombocytopenic purpura (ITP), autoimmune uveitis, or autoimmune thrombocytopenia.

In the present specification, the term "allergy" refers to an immune reaction occurring excessively against a specific non-self antigen, and is a disease in which an immune reaction occurs against an "allergen". An "allergen" refers to an antigen that can react with an antibody of a subject having an allergic disease. Examples thereof include, but are not limited to, allergens derived from pollen of trees (acacia, alder, velvet ash, European beech, birch, maple, mountain cedar, red cedar, poplar, Japanese cypress, American elm, Chinese elm, Douglas fir, rubber tree, eucalyptus tree, hackberry, hickory, American linden, sugar maple, mesquite, paper mulberry, Quercus, olive, pecan, pepper, pine, privet, Russian olive, American sycamore, tree of heaven, black walnut, black willow, and the like), allergens derived from pollen of grasses and plants (cotton, Bermuda grass, Kentucky bluegrass, brome, corn, meadow fescue, Johnson grass, oat, orchard grass, redtop, ryegrass, rice, sweet vernal grass, timothy, amaranth, goosefoot, cocklebur, dock, goldenrod, kochia, white goosefoot, marigold, nettle, barnyard grass, plantain, giant ragweed, ragweed, false ragweed, Russian thistle, mugwort, broom, sheep sorrel, and the like), insect-derived allergens (silkworms, mites, honeybees, wasps, ants, cockroaches, and the like), fungus-derived allergens (Alternaria, Aspergillus, Botulinus, Candida, Cephalosporium, Curvularia, Epicoccum, Epidermophyton, Fusarium, Helminthosporium, Cladosporium herbarum, Mucor, Penicillium, Phoma, Pullularia pullulans, Rhizopus, and the like), allergens derived from animal hair (dogs, cats, birds, and the like), allergen proteins derived from house dust, food-derived allergens (OVA and the like), and the like. Representative diseases of "allergy" include atopic dermatitis, allergic rhinitis (pollinosis and the like), allergic conjunctivitis, allergic gastroenteritis, bronchial asthma, childhood asthma, food allergy, drug allergy, urticaria, and the like.

### (Preferred embodiments)

Preferred embodiments of the present disclosure will be described below. The embodiments provided below are provided for a better understanding of the present disclosure, and the scope of the present disclosure should not be limited to the following description. Therefore, it is apparent that those skilled in the art can appropriately make modifications within the scope of the present disclosure in view of the description in the present specification. In addition, the following embodiments of the present disclosure can be used alone or in combination.

In one aspect, the present disclosure provides immune cells such as T cells with enhanced glucose uptake ability, a cell population containing such cells, a pharmaceutical composition containing such cells or the cell population, and other related technologies.

In one aspect of the present disclosure, T cells with enhanced glucose uptake ability are provided. It has been unexpectedly shown that T cells modified to express a glucose transporter and/or having enhanced expression of a glucose transporter exert remarkable effects on diseases such as cancer, autoimmune diseases, allergies, and infectious diseases when applied in the form of CAR-T cells, TCR-T cells, and the like (see FIG. 16). It is advantageous that the cells of the present disclosure have enhanced glucose uptake ability. In addition, the cells of the present disclosure preferably have an effector function after being introduced into a place where they are to function, particularly into the body. Such cells include, but are not limited to, T_{eff}, and may also include precursor cells of T_{eff}, for example, lymphoid progenitor cells, Tnv, Tscm, Temra, and the like.

In one embodiment, the present disclosure may be an effector T cell that includes a chimeric antigen receptor (CAR) and is modified to express a glucose transporter and/or has enhanced expression of a glucose transporter. In another embodiment, the present disclosure may be a cell population containing effector T cells that include a chimeric antigen receptor (CAR) and are modified to express a glucose transporter and/or have enhanced expression of a glucose transporter. The present disclosure provides "effector T cells", which could not be provided by the related art. The effector T cell referred to in this embodiment means an immune cell (for example, a T cell) that exerts a cytotoxic effect on target cells (as described elsewhere in the present specification). As described elsewhere in the present specification, effector functions include cytotoxic activity, cytokine production, and division and proliferation resulting from signals mediated by TCR or CAR, and one important function as an effector T cell is to exert a cytotoxic effect on target cells.

On the other hand, the related art has not provided effector T cells capable of exerting a cytotoxic effect on target cells. Rather than merely not suggesting this, the related art has no examples in which cure was confirmed by experiments such as rechallenge, and, for example, there are also examples in which nodules remain, and there are many examples showing that cure has not been achieved. Here, although various therapeutic agents for cancer are known, most conventional therapeutic agents merely reduce tumors and have not been able to eradicate them. This is because they do not have the function of exerting a cytotoxic effect on target cells as in the present disclosure. In the related art, a tumor-suppressing effect may be observed due to the effects of cytokines and the like, and most anticancer agents have only such indirect effects. In the related art, unlike the present disclosure, in an in vivo model, even if a tumor is said to be suppressed, the related art does not exert a cytotoxic effect on target cells; rather, the tumor remains, and it can be said that the related art does not have a function of exerting a cytotoxic effect on target cells. The present disclosure exhibits an effect different from these related art techniques in this respect.

More specifically, in an embodiment of the present disclosure, chimeric antigen receptor T (CAR-T) cell therapy has been unable to demonstrate efficacy against solid cancers, but the present disclosure also solves this problem. Here, the present inventors found that dysfunction of CAR-T cells in solid cancers is caused by glucose deficiency in the tumor microenvironment (TME), and that metabolic supplementation thereof significantly improves the antitumor effect of CAR-T cells. Massive consumption of glucose by cancer cells decreases glucose levels in the TME of solid cancers, and consequently impairs CAR-T cells. In a specific embodiment of the present disclosure, the present inventors found that, when GLUTs, which are glucose transporters, are expressed in CAR-T cells, cytokine production and killing activity are restored, or effector function is improved. The present disclosure provides a treatment method for various solid cancers that could not be radically cured even by combination therapy using chemotherapeutic agents, radiation, and the like. In certain solid cancers, a metabolically harsh TME is present, which impairs the function of effector T cells including CD8⁺ T cells, and CAR-T cells are adoptively transferred into the TME. The present disclosure is particularly effective in examples in which nervous system cells such as neurons and glial cells require glucose as an energy source. Furthermore, certain aggressive cancer cells consume more glucose than normal cells due to aerobic glycolysis, which is a characteristic metabolism of cancer cells known as the Warburg effect, leading to depletion of glucose in the TME. On the other hand, naive T cells shift energy production from oxidative phosphorylation and fatty acid oxidation to aerobic glycolysis upon activation in order to meet increased energy demand. Therefore, intense glucose-mediated metabolic competition occurs in the TME between activated T cells and cancer cells. Cancer cells can survive and continuously proliferate while taking up sufficient glucose in the TME, whereas activated T cells have impaired function due to failure in metabolic competition. Expression of GLUT at an equivalent or higher level by CAR-T cells that exogenously express GLUT or have enhanced endogenous expression of GLUT may enable complete competition with cancer cells.

More specifically, the following is observed as a preferable, non-limiting embodiment of the present disclosure. That is, despite the clinical success of chimeric antigen receptor T (CAR-T) cell therapy in hematologic malignancies, application to refractory solid tumors including glioblastoma (GBM) has not yet been successful, and clinical trials of CAR-T cells against solid cancers have so far failed to demonstrate efficacy, but the present disclosure also solves this problem. Here, the present inventors found that dysfunction of CAR-T cells in GBM is caused by glucose deficiency in the tumor microenvironment (TME), and that metabolic supplementation thereof significantly improves the antitumor effect of CAR-T cells. Massive consumption of glucose by cancer cells decreases glucose levels in the TME of GBM, and consequently impairs CAR-T cells. In a preferred embodiment of the present disclosure, when GLUT3, which is a high-affinity glucose transporter, was stably expressed in CAR-T cells, cytokine production and killing activity were restored. Glioblastoma (GBM) is a devastating malignant brain tumor, with an annual incidence of 3.19 per 100,000 people. Since the introduction in 2005 of combination therapy consisting of temozolomide and radiation, and the development of tumor treating fields therapy (Novo TTF) for primary GBM, the prognosis of GBM patients in clinical practice has improved. However, the 5-year overall survival rate of GBM remains below 20%. In light of the clinical success of chimeric antigen receptor T cell (CAR-T cell) therapy in hematologic malignancies, several preclinical and clinical trials of CAR-T cell therapy for GBM have been actively investigated. Some patients treated in clinical trials show promising results, such as infiltration of CAR-T cells into the tumor microenvironment (TME) and reduction in tumor burden, but most patients do not respond to CAR-T cell therapy. GBM has a metabolically harsh TME, which impairs the function of effector T cells including CD8⁺ T cells and adoptively transferred CAR-T cells in the TME. In the brain, nervous system cells such as neurons and glial cells require glucose as an energy source, and the brain consumes 25% of whole-body glucose-derived energy. Furthermore, aggressive GBM cells consume three times more glucose than normal neurons due to aerobic glycolysis, which is a characteristic metabolism of cancer cells known as the Warburg effect. This leads to depletion of glucose in the TME of GBM. On the other hand, naive T cells shift energy production from oxidative phosphorylation and fatty acid oxidation to aerobic glycolysis upon activation in order to meet increased energy demand. Therefore, intense glucose-mediated metabolic competition occurs in the TME of GBM between activated T cells and cancer cells. In a preferred embodiment, GLUT3, which is encoded by SLC2A3 and physiologically expressed in neurons and glial cells, has a 5-fold higher affinity for glucose than GLUT1, which is the main glucose transporter expressed in T cells. As a result, GBM cells expressing GLUT3 can survive and continuously proliferate while taking up sufficient glucose in the TME, whereas activated T cells have impaired function due to failure in metabolic competition. In this preferred embodiment, considering substantial glucose uptake by GBM cells via high-affinity GLUT3, equivalent or higher expression of GLUT3 by CAR-T cells may enable complete competition with GBM cells. In one preferred embodiment of the disclosure, a construct in which SLC2A3 is linked to CAR is provided in order to induce expression of GLUT3 in CAR-T cells.

### (Cell population)

In one aspect of the present disclosure, a cell population containing immune cells such as T cells with enhanced glucose uptake ability is provided. It has been unexpectedly shown that, when at least some of the T cells contained in this cell population are modified to express a glucose transporter and/or have enhanced expression of a glucose transporter, remarkable effects on diseases such as cancer, autoimmune diseases, allergies, and infectious diseases are exerted when applied as CAR-T cells, TCR-T cells, and the like (see FIG. 16 and the like). It is advantageous that the cell population of the present disclosure has enhanced glucose uptake ability. In addition, the cell population of the present disclosure preferably contains cells that have an effector function after being introduced into a place where they are to function, particularly into the body. Such cells include, but are not limited to, T_{eff}, and may also include precursor cells of T_{eff}, for example, lymphoid progenitor cells, Tnv, Tscm, Temra, and the like.

In an embodiment of the present disclosure, in a cell population containing a cell population containing T cells as described above, it is advantageous that the cell population contains T cells having the features of the present disclosure (for example, enhancement or expression of GLUT, an effector function, or both) in an amount of about 10% or more in the cell population. In an embodiment, in the cell population of the present disclosure, the proportion of T cells having the features of the present disclosure may be about 5% or more, about 10% or more, about 15% or more, about 20% or more, about 25% or more, about 30% or more, about 35% or more, about 40% or more, about 45% or more, about 50% or more, about 55% or more, about 60% or more, about 65% or more, about 70% or more, about 75% or more, about 80% or more, about 85% or more, about 90% or more, about 95% or more, about 97% or more, or about 99% or more.

In one embodiment, the cell population of the present disclosure contains T cells having a property of exhibiting an effector function when introduced into the body.

In one embodiment, in the cell population of the present disclosure, the T cells include a chimeric antigen receptor (CAR) or a T cell receptor (TCR), and preferably include a chimeric antigen receptor (CAR).

In one embodiment, in the cell population of the present disclosure, the T cells are modified to express a glucose transporter and/or have enhanced expression of a glucose transporter.

In one embodiment, in the cell population of the present disclosure, the T cells have an effector function.

In one embodiment, the cell population of the present disclosure contains T_{eff} and/or precursor cells of T_{eff}. Preferably, the cell population (or precursor cells of T_{eff}) contains at least one type of cell such as lymphoid progenitor cells, Tnv, Tscm, Tcm, or Temr.

In one embodiment, in the cell population of the present disclosure, the CAR is expressed in the T cells.

In one embodiment, in the cell population of the present disclosure, the glucose transporter includes GLUT1, GLUT2, GLUT3, and GLUT4, and is preferably GLUT3.

In one embodiment, in the cell population of the present disclosure, the T cells are human T cells.

In one embodiment, the cell population of the present disclosure is provided as a medicament or pharmaceutical composition. The use thereof may be for prevention or treatment of cancer, for cure of cancer, or for prevention of cancer metastasis or prevention of cancer recurrence. In a specific embodiment, the cell population of the present disclosure may be for preventing or treating cancer so that the cancer does not recur.

### <Glucose transporter>

Cancer cells reprogram their metabolic systems to favor their own cell proliferation, actively use the glycolytic pathway, which has low efficiency in ATP production, even in the presence of oxygen, and increase glucose uptake and lactic acid production (Warburg effect). Therefore, because cancer cells consume and deplete a large amount of glucose, T_{eff}-type tumor-specific T cells, which require glucose as an energy source, have a decreased cellular Ca²⁺ concentration and become starved and exhausted when they infiltrate into a tumor, even if they receive a signal through TCR.

Accordingly, in an embodiment of the present disclosure, T cells and cell populations genetically engineered to express a glucose transporter are provided in order to promote aerobic glycolysis and enhance competitiveness and/or fitness of immune cells in a tumor microenvironment (TME) in which glucose is depleted.

In an embodiment, examples of the glucose transporter include GLUT1, GLUT2, GLUT3, and GLUT4, and among these, the glucose transporter having high affinity for glucose is GLUT3. Therefore, in a preferred embodiment, the glucose transporter used in the cells and cell population of the present disclosure is GLUT3.

GLUT3 was first identified in the mouse brain and was initially defined as a neuronal glucose transporter. Thereafter, GLUT3 was shown to be expressed also in other cells that require glucose, such as mouse sperm, which secure energy necessary for motility, and blastocysts, which are important for post-implantation development. Furthermore, GLUT3 is expressed in immune cells such as lymphocytes, monocytes, macrophages, and platelets, and is usually stored in intracellular vesicles and, upon activation, moves to the cell surface to maintain a metabolic switch. More recently, it has been suggested that T cells, particularly CD8⁺ T cells, depend not only on GLUT1 but also on GLUT3 for glucose uptake because GLUT3 is highly expressed during differentiation and activation.

When a glucose transporter such as GLUT3 is activated in T_{eff}, enhancement of effector functions such as cytotoxic activity and cytokine production ability is observed due to glucose uptake.

In an embodiment of the present disclosure, the T cells of the present disclosure or the T cells contained in the cell population may include effector cells.

In an embodiment of the present disclosure, the T cells of the present disclosure and the T cells contained in the cell population may advantageously be human T cells.

### <Chimeric antigen receptor (CAR)>

In another aspect of the present disclosure, provided are a T cell with enhanced glucose uptake ability or a cell population containing such cells, in which the T cell contains a chimeric antigen receptor (CAR), and the T cell is modified to express a glucose transporter and/or has enhanced expression of a glucose transporter. The T cell of the present disclosure, or the T cells contained in the cell population, may contain the CAR as a protein, or may contain a nucleic acid molecule expressing the CAR, as long as the CAR can exert a function as a CAR. In a representative embodiment, the CAR of the present disclosure may be expressed in T cells.

The CAR disclosed in the present specification includes at least one extracellular domain capable of binding to an antigen, at least one transmembrane domain, and at least one intracellular domain.

A chimeric antigen receptor (CAR) is an artificially constructed hybrid protein or polypeptide containing an antigen-binding domain of an antibody (for example, a single-chain variable fragment (scFv)) linked to a T cell signaling domain via a transmembrane domain. A feature of CAR is that CAR redirects the specificity and reactivity of T cells toward a selected target by utilizing the antigen-binding properties of a monoclonal antibody independently of MHC. MHC-independent antigen recognition can provide T cells expressing CAR or T cells in a cell population with the ability to recognize an antigen independently of antigen processing, and can avoid immune escape of tumors.

The intracellular T cell signaling domain of CAR may include, for example, a T cell receptor signaling domain, a T cell co-stimulatory signaling domain, or both. The T cell receptor signaling domain refers to a portion of CAR including an intracellular domain of a T cell receptor, for example, an intracellular portion of a CD3 zeta protein. The co-stimulatory signaling domain refers to a portion of CAR including an intracellular domain of a co-stimulatory molecule, which is a cell surface molecule other than an antigen receptor or a ligand thereof, required for an efficient response of lymphocytes to an antigen.

### <T cell receptor (TCR)>

In another aspect of the present disclosure, provided are a T cell with enhanced glucose uptake ability or a cell population containing such cells, in which the T cell includes a T cell receptor (TCR), and the T cell is modified to express a glucose transporter and/or has enhanced expression of a glucose transporter. As long as the TCR can exert a function as a TCR, the T cell of the present disclosure or the T cells contained in the cell population may contain the TCR as a protein or may contain a nucleic acid molecule expressing the TCR. In a representative embodiment, the TCR of the present disclosure may be expressed in T cells.

In the present specification, TCR is a heterodimeric receptor molecule consisting of two TCR polypeptide chains, and includes αβ-type TCR expressed by ordinary T cells and γδ-type TCR having special functions. The α- and β-chain TCR molecules form a complex with a plurality of CD3 molecules (CD3ζ chain, CD3ε chain, CD3γ chain, and CD3δ chain), transmit intracellular signals after antigen recognition, and initiate various immune responses. Endogenous antigens, such as viral antigens that have proliferated intracellularly in association with viral infection and cancer antigens derived from cancer cells, are presented as antigen peptides on MHC class I molecules. In addition, antigens derived from foreign microorganisms are taken up by antigen-presenting cells by endocytosis, and after being processed, are presented on MHC class II molecules. These antigens are recognized by TCRs expressed by CD8+ T cells or CD4+ T cells, respectively. It is also known that costimulatory molecules such as CD28, ICOS, and OX40 molecules are important for stimulation mediated by TCR molecules. With respect to αβ-type TCR, the respective gene products of α and β can exhibit specificity through a unique combination. TCR can be modified as appropriate and may include a T cell receptor signaling domain, a T cell co-stimulatory signaling domain, or both. The T cell receptor signaling domain includes an intracellular domain of a T cell receptor, for example, an intracellular portion of a CD3 zeta protein. The co-stimulatory signaling domain includes an intracellular domain of a co-stimulatory molecule, which is a cell surface molecule other than an antigen receptor or a ligand thereof, required for an efficient response of lymphocytes to an antigen.

### (Extracellular domain)

In an embodiment, the CAR used in the T cell disclosed in the present specification or the T cells contained in the cell population includes an antigen-binding domain or a portion thereof. The antigen-binding domain or a portion thereof can be appropriately selected according to the type and number of ligands on the surface of target cells, and, for example, the antigen-binding domain may be selected to recognize a ligand that acts as a cell surface marker on a target cell associated with a specific disease state. Therefore, examples of cell surface markers that may act as ligands for the antigen-binding domain in the CAR of the present disclosure include tissue-specific markers, cytoma-specific markers, and those associated with viral, bacterial, and parasitic infections, autoimmune diseases, and cancer cells.

The extracellular binding domain of CAR may be constituted by a single-chain variable fragment (scFv) obtained by fusing variable heavy and light regions of a mouse or humanized monoclonal antibody. Alternatively, an scFv derived from Fab (obtained, for example, from a Fab library, not from an antibody) may be used. The scFv can be fused to a transmembrane domain and then to an intracellular signaling domain.

In an embodiment, the antigen-binding domain portion of the CAR of the present disclosure can target antigens including: (1) alloantigens including MHC class I, MHC class II, and the like; (2) extracellular autoantigens including thyroid stimulating hormone receptor (TSHR), desmoglein 3 (DSG3), Cytokeratin8, and the like; (3) foreign antigens including Gliadin, Ara h2, and the like; and (4) targeting molecules including CD4, CD8, CD19, BCMA, CD68, mesothelin (MSLN), mucosal vascular addressin cell adhesion molecule 1 (MadCam1), and the like, but antigens that can be targeted by the antigen-binding domain portion of the CAR of the present disclosure are not limited thereto.

In an embodiment, depending on a desired antigen to be targeted, the CAR of the present disclosure can be modified to include an antigen-binding domain specific to the desired antigen target. For example, when CD19 is a target antigen, an antibody against a cancer antigen such as CD19 may be used as an antigen-binding domain in the CAR. Non-limiting examples of cancer antigens include CD19, CD20, CD30, CD33, CD38, CD133, BCMA, TEM8, EpCAM, ROR1, folate receptor, CD70, MAGE-1, MAGE-2, MAGE-3, MAGE A-10, MAGE-C2, MAGE-A12, CEA, tyrosinase, midkine, BAGE, CASP-8, P-catenin, CA-125, CDK-1, ESO-1, gp75, MART-1, MUC-1, MUM-1, p53, PAP, PSA, PSMA, ras, trp-1, HER-2, TRP-1, TRP-2, IL13Ralpha, IL13Ralpha2, AIM-2, AIM-3, NY-ESO-1, C9orfl l2, SART1, SART2, SART3, BRAP, RTN4, GLEA2, TNKS2, KIAA0376, ING4, HSPH1, C13orf24, RBPSUH, C6orfl53, NKTR, NSEP1, U2AF1L, CYNL2, TPR GOLGA, BMI1, COX-2, EGFRvIII, EZH2, LICAM, Livin, LivinP, MRP-3, Nestin, OLIG2, ART1, ART4, B cycling, Grill, Cav-1, cathepsin B, CD74, E-Cadherin, EphA2/Eck, Fra-1/Fosl 1, GAGE-1, ganglioside/GD2, GnT-V, pl, 6-N, Ki67, Ku70/80, PROXI, PSCA, SOXIO, SOX11, Survivin, phCG, WT1, mesothelin, Melan A, NY-BR-1, NY-CO-58, MN (gp250), telomerase, SSX-2, PRAME, PLK1, VEGF-A, VEGFR2, and Tie-2. In some embodiments, the effector T cells disclosed in the present specification are engineered to express one or more CARs in order to recognize one or more antigens.

### (Transmembrane domain)

The CAR used in the T cell disclosed in the present specification or the T cells contained in the cell population can include one or more transmembrane domains fused to the extracellular domain.

In an embodiment, a linker domain derived from the extracellular domain may be linked to the transmembrane domain. The transmembrane domain may be natural or synthetic, and a natural transmembrane domain may be derived from any membrane-bound or transmembrane protein. The transmembrane region particularly used in the present disclosure may be derived from the alpha, beta, or zeta chain of a T cell receptor, CD28, CD3 epsilon, CD45, CD4, CD5, CD8, CD9, CD16, CD22, CD33, CD37, CD64, CD80, CD86, CD134, CD137, CD154, CD271, TNFRSF19, and the like.

In an embodiment, in the CAR used in the T cell disclosed in the present specification or in the T cells contained in the cell population, a spacer domain may also be disposed between the extracellular domain and the transmembrane domain, or between the intracellular domain and the transmembrane domain. The spacer domain can preferably have a sequence that promotes binding of the CAR to an antigen and enhances signal transduction into cells.

### (Intracellular domain)

The cytoplasmic signaling domain of CAR (or intracellular signaling domain) is involved in activation of at least one normal effector function of an immune cell expressing the CAR. The intracellular signaling domain refers to a portion of a protein that transmits an effector function signal and instructs a cell expressing the CAR to perform a specialized function. The intracellular signaling domain may include any complete, mutant, or truncated portion of an intracellular signaling domain of a given protein that is sufficient to transmit a signal that elicits or blocks an immune cell effector function.

In an embodiment, examples of the intracellular signaling domain used in CAR include cytoplasmic signaling sequences of a T cell receptor (TCR) and co-receptors that elicit signal transduction after antigen receptor binding.

### (Alloantigens, allergens, and haptens related to rejection)

The CAR used in the T cell disclosed in the present specification or in the T cells contained in the cell population can include those related to alloantigens associated with rejection, allergens, and haptens.

### <Pharmaceutical use of T cells or T cells contained in cell population>

In one aspect of the present disclosure, provided is a pharmaceutical composition containing a T cell with enhanced glucose uptake ability or a cell population containing such T cells, in which the T cell is modified to express a glucose transporter and/or has enhanced expression of a glucose transporter. In another aspect of the present disclosure, provided is a pharmaceutical composition containing a T cell with enhanced glucose uptake ability or a cell population containing such T cells, in which the T cell contains a chimeric antigen receptor (CAR), a T cell receptor (TCR), or the like, and the T cell is modified to express a glucose transporter and/or has enhanced expression of a glucose transporter. In an embodiment of the present disclosure, the T cell in the present disclosure or the T cells contained in the cell population can have one or more of the features of the T cells described above.

The cells, cell populations, and the like of the present disclosure can be used in immunotherapy. Immunotherapy is considered effective against diseases in which lesions having antigenicity occur (for example, cancer, autoimmune diseases, allergies, infectious diseases, and the like), and diseases in which an abnormal immune response to a specific antigen is involved in onset or progression of the pathology. For example, the cells and the like of the present disclosure can be used for treatment, therapy, or prevention of autoimmune diseases, allergic diseases, or graft-versus-host disease (GVHD), rejection, or engraftment failure at the time of transplantation. Examples of autoimmune diseases include, but are not limited to, rheumatoid arthritis (RA), Sjögren's syndrome, systemic lupus erythematosus (SLE), antiphospholipid syndrome, polymyositis/dermatomyositis, systemic sclerosis, mixed connective tissue disease, vasculitis syndrome, type I diabetes, Graves' disease, Hashimoto's disease, idiopathic Addison's disease, autoimmune hepatitis, Goodpasture's syndrome, glomerulonephritis, autoimmune hemolytic anemia (AIHA), autoimmune thrombocytopenic purpura, autoimmune neutropenia, myasthenia gravis, pemphigus, vitiligo, and idiopathic azoospermia. Examples of allergic diseases include, but are not limited to, pollinosis, allergic rhinitis, bronchial asthma, and atopic dermatitis. In addition, treatment or prevention with the cells, cell populations, and the like of the present disclosure can be used for diseases in which an abnormal immune response to a specific antigen is involved in onset or progression of the pathology.

In a specific embodiment, the present disclosure provides such immunotherapy, and the immunotherapy includes TCR-T therapy or CAR-T therapy. In the present specification, the term "TCR-T therapy" refers to cell therapy using modification of a T cell receptor (TCR), and refers to, for example, therapy used for cancer treatment. In the present specification, the term "CAR-T therapy" refers to gene and cell therapy in which a chimeric antigen receptor (CAR) (for example, one genetically engineered to overcome a tumor immune evasion mechanism) is genetically introduced into T cells of a patient, and the T cells are expanded and cultured ex vivo and then infused into the patient.

The cells, cell populations, and the like of the present disclosure may be appropriately combined with other cancer treatments and may be used as combination therapy. Typically, they may be administered in combination with one further drug or a plurality of further drugs. Alternatively, the combination therapy may be a combination with radiation therapy. The one further drug or the plurality of further drugs may be any chemotherapeutic agent or may include an immune checkpoint inhibitor. Alternatively, other cancer treatments used in the combination therapy include, but are not limited to, other cancer immunotherapies (for example, immune checkpoint inhibitors), thermotherapy, surgical procedures, and the like.

In one aspect, provided is a therapeutic agent containing the T cells or cell population of the present disclosure, in which a disease of a subject is diagnosed, and an appropriate CAR, TCR, or the like contained in the T cells or the cell population is selected based on the diagnosis.

In another aspect, the present disclosure provides a pharmaceutical composition containing any of the immune cells described in the present specification (for example, T cells such as effector T cells) or a cell population containing such cells, and a pharmaceutically acceptable carrier. When the immune cells express a polypeptide such as CAR or TCR, the pharmaceutical composition may further contain an Fc-containing therapeutic agent such as a therapeutic antibody or an Fc fusion protein. The Fc-containing therapeutic agent can bind to a target antigen, such as one on immune cells specific for a tumor antigen, a pathogen antigen, or self-antigen. The pathogen antigen may be a bacterial antigen, viral antigen, or fungal antigen.

In an embodiment, the Fc-containing therapeutic agent may be a therapeutic antibody including, but not limited to, adalimumab, ado-trastuzumab emtansine, alemtuzumab, basiliximab, bevacizumab, belimumab, brentuximab, canakinumab, cetuximab, certolizumab, daclizumab, denosumab, dinutuximab, eculizumab, efalizumab, epratuzumab, gemtuzumab, golimumab, hu14.18K322A, ibritumomab, infliximab, ipilimumab, labetuzumab, muromonab, natalizumab, obinutuzumab, ofatumumab, omalizumab, palivizumab, panitumumab, pertuzumab, ramucirumab, ranibizumab, rituximab, tocilizumab, trastuzumab, tositumomab, ustekinumab, mogamulizumab, and vedolizumab.

Furthermore, the present disclosure provides a kit containing: (i) a first pharmaceutical composition containing any of the T cells described in the present specification or a cell population containing such T cells, and a pharmaceutically acceptable carrier; and (ii) the Fc-containing therapeutic agent described in the present specification and a pharmaceutically acceptable carrier.

In another aspect, the present disclosure provides a method for inhibiting cells expressing a target antigen in a subject (for example, reducing the number of such cells, inhibiting cell proliferation, and/or suppressing cellular activity), the method including administering the cells, cell population, and/or pharmaceutical composition of the present disclosure to the subject. In an embodiment, at least some of the cells expressing the target antigen may be in a low-glucose environment.

In an embodiment, the subject treated by the method of the present disclosure may be a human patient suffering from cancer, for example, carcinoma, lymphoma, sarcoma, blastoma, or leukemia. Examples of target cancers include, but are not limited to, B-cell-derived cancer, breast cancer, gastric cancer, neuroblastoma, osteosarcoma, lung cancer, skin cancer, prostate cancer, colon cancer, renal cell carcinoma, ovarian cancer, rhabdomyosarcoma, leukemia, mesothelioma, pancreatic cancer, head and neck cancer, retinoblastoma, glioma, glioblastoma, liver cancer, and thyroid cancer. Examples of B-cell-derived cancer include B-cell acute lymphoblastic leukemia, B-cell chronic lymphocytic leukemia, and B-cell non-Hodgkin lymphoma.

In addition to treating a target disease or disorder such as cancer or an infectious disorder, use of the T cells or cell population of the present disclosure for producing a medicament for a medical treatment of interest is also within the scope of the present disclosure.

In an embodiment, a pharmaceutical composition containing effector T cells including the chimeric antigen receptor (CAR) of the present disclosure can be used in cell therapy. In cell therapy, T cells including the chimeric antigen receptor (CAR), T cell receptor (TCR), or the like of the present disclosure, or a cell population containing such T cells, may be infused into a subject in need thereof as a pharmaceutical composition or as a preparation of a therapeutically effective cell population expressing the CAR, T cell receptor (TCR), or the like of the present disclosure. The infused T cells or cell population containing such T cells in the subject may treat a target disease or disorder of the subject, such as cancer or an infectious disorder. The subject may be the same subject from which the cells were obtained (autologous cell therapy), or the cells may be derived from another subject of the same species (allogeneic cell therapy).

In an embodiment, T cells including the CAR, (TCR), or the like of the present disclosure, or a cell population containing such T cells, can be formulated for administration to a subject using techniques known to those skilled in the art. In an embodiment, a preparation containing therapeutically effective T cells including the CAR, (TCR), or the like of the present disclosure, or a cell population containing such T cells, may contain a pharmaceutically acceptable excipient (carrier or diluent). The excipient contained in the preparation has different purposes depending on, for example, the nature of the antigen-binding domain of the CAR of the present disclosure. Non-limiting examples of commonly used excipients include the following: saline, buffered saline, dextrose, water for injection, glycerol, ethanol, and combinations thereof, stabilizers, solubilizers and surfactants, buffers and preservatives, tonicity agents, bulking agents, and lubricants.

In the present specification, when a gene name and a product thereof are indicated, they may refer to both a gene and a protein when expressed in all capital letters, unlike normal usage. For example, FOXP3 gene and FOXP3 protein may be referred to separately, and when expressed as FoxP3, it indicates both the concept and the actual entity (as a whole) of the gene or the protein.

A preparation containing therapeutically effective T cells including the CAR of the present disclosure, or a cell population containing such T cells, may be administered to a subject using methods and techniques known to those skilled in the art. Examples thereof include, but are not limited to, intravenous injection. Other methods include, but are not limited to, intratumoral, intradermal, subcutaneous, intramuscular, intraperitoneal, intraarterial, intramedullary, intracardiac, intraarticular (joint), intrabursal (synovial fluid area), intracranial, intraspinal, and intrathecal (cerebrospinal fluid) administration.

### (General technique)

The molecular biological techniques, biochemical techniques, and microbiological techniques used in the present specification are well known and commonly used in the art, and are described in, for example, Sambrook J. et al.

(1989). Molecular Cloning: A Laboratory Manual, Cold Spring Harbor and its 3rd Ed. (2001); Ausubel, F.M. (1987). Current Protocols in Molecular Biology, Greene Pub. Associates and Wiley-Interscience; Ausubel, F.M. (1989). Short Protocols in Molecular Biology: A Compendium of Methods from Current Protocols in Molecular Biology, Greene Pub. Associates and Wiley-Interscience; Innis, M.A. (1990). PCR Protocols: A Guide to Methods and Applications, Academic Press; Ausubel, F.M. (1992). Short Protocols in Molecular Biology: A Compendium of Methods from Current Protocols in Molecular Biology, Greene Pub. Associates; Ausubel, F.M. (1995). Short Protocols in Molecular Biology: A Compendium of Methods from Current Protocols in Molecular Biology, Greene Pub. Associates; Innis, M.A. et al. (1995). PCR Strategies, Academic Press; Ausubel, F.M. (1999). Short Protocols in Molecular Biology: A Compendium of Methods from Current Protocols in Molecular Biology, Wiley, and annual updates; Sninsky, J.J. et al. (1999). PCR Applications: Protocols for Functional Genomics, Academic Press, the special volume of Experimental Medicine, "Experimental Methods for Gene Introduction & Expression Analysis", YODOSHA CO., LTD., 1997, and the like, and the relevant parts thereof (which may be the entirety thereof) are incorporated herein by reference.

For DNA synthesis techniques and nucleic acid chemistry for producing artificially synthesized genes, gene synthesis or fragment synthesis services such as GeneArt, GenScript, and Integrated DNA Technologies (IDT) can also be used. In addition, such techniques and chemistry are described in, for example, Gait, M.J. (1985). Oligonucleotide Synthesis: A Practical Approach, IRL Press; Gait, M.J. (1990). Oligonucleotide Synthesis: A Practical Approach, IRL Press; Eckstein, F. (1991). Oligonucleotides and Analogues: A Practical Approach, IRL Press; Adams, R.L. et al. (1992). The Biochemistry of the Nucleic Acids, Chapman & Hall; Shabarova, Z. et al. (1994). Advanced Organic Chemistry of Nucleic Acids, Weinheim; Blackburn, G.M. et al. (1996). Nucleic Acids in Chemistry and Biology, Oxford University Press; Hermanson, G.T. (I996). Bioconjugate Techniques, Academic Press, and the like, and the relevant parts thereof are incorporated herein by reference.

In the present specification, "or" is used when "at least one or more" of the items listed in the text can be employed. The same applies to "or". When explicitly described in the present specification as "within the range" of "two values", the range also includes the two values themselves.

References such as scientific literature, patents, and patent applications cited in the present specification are incorporated herein by reference in their entirety to the same extent as if each were specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described in the present specification, but is limited only by the claims.

### Examples

In the present Example, various functions of T cells with enhanced glucose uptake ability were examined. For reagents, the specific products described in the Examples were used; however, equivalent products from other manufacturer (Sigma-Aldrich, Wako Pure Chemical, NACALAI TESQUE, INC., R&D Systems, USCN Life Science INC, or the like) may also be substituted.

### (Example 1)

### Methods and materials

### Preparation of GLUT3 3C10-CAR construct and preparation of viral vector

An SLC2A3 sequence (GLUT3), with a P2A sequence disposed immediately upstream thereof, was inserted into an anti-EGFRvIII CAR backbone plasmid (3C10 CAR-Katushka2S) having a structure of CD8 leader sequence-anti-EGFRvIII single-chain variable region (scFv)-CD8 hinge-CD8 transmembrane portion (TM)-CD28 intracellular domain (ICD)-4-1BB ICD-CD3z-P2A-Katushka2S, and a 3C10 CAR-GLUT3-Katushka2S lentiviral vector was constructed (FIG. 1). The lentiviral vector was transfected into 293T cells together with packaging vectors psPAX2 (Addgene #12260) and pMD2.G (Addgene #12259), and lentivirus was obtained from the supernatant.

### Gene transfer

Peripheral blood mononuclear cells from healthy subjects were separated from heparinized whole blood by the Ficoll method (Ficoll-Paque PLUS, GE Healthcare), stimulated with anti-CD3/28 beads (Dynabeads T-Activator CD3/CD28, Veritas), and CAR gene transfer was performed at an MOI=4 on day 1 after stimulation. The cells were cultured in the presence of IL-2 30 U/ml and were cryopreserved on days 8 to 12 or directly subjected to each evaluation.

### Immunostaining

1 × 10⁶ GLUT3 3C10 CAR-T cells were fixed with 4% PFA and permeabilized with methanol. Blocking was performed with 3% BSA for 1 hour, and the cells were incubated with an anti-GLUT3 antibody (ab15311) for 2 hours. The cells were washed with 0.05% PBST and incubated with an anti-rabbit IgG antibody (Alexa flour 488) for 1 hour. The cells were washed with 0.05% PBST, and nuclear staining was performed with DAPI. After staining, the cells were adhered to a slide glass using Cytospin (Thermo Fisher Scientific), mounted using VECTASHIELD (VECTOR LABORATORIES), and observed using Keyence XZ-800.

### Phenotype analysis (FCM)

The antibodies used for FCM analysis are shown in Table 1.

**[Table 1]**

| Antibody | Company | Product number |
|---|---|---|
| Anti-human CD3-BV786(SK7) | BD Biosciences | 563800 |
| Anti-human CD4-AF700(SK3) | BD Biosciences | 566909 |
| Anti-human CD4-BUV805(SK3) | BD Biosciences | 612888 |
| Anti-human CD45RA-PerCP-Cy5.5(HI100) | BD Biosciences | 563429 |
| Anti-human CD27-BV711(O323) | Biolegend | 302834 |
| Anti-human CCR7-R718(2-L1-A) | BD Biosciences | 751859 |
| Anti-human CD279(PD-1)-BV421(NIH4) | BD Biosciences | 564323 |
| Anti-human LAG3-FITC(17B4) | Enzo | ALX-804-806F-C100 |
| Anti-human TIM3-BV605(F38-2E2) | Biolegend | 345018 |
| Anti-human CD25-PE(M-A251) | BD Biosciences | 560989 |
| Anti-human CCR4-PeCy7(L291H4) | Biolegend | 359410 |
| Anti-human CD183(CXCR3)-APC(G025H7) | Biolegend | 353708 |
| Anti-human IFNr-AF488(4S.B3) | Biolegend | 502515 |
| Anti-human TNFa-APC(MAb11) | Invitrogen | 17-7349-82 |
| Anti-human IL2-PE/Cy7(MQ1-17H12) | Invitrogen | 25-7029-42 |
| Fixable Viability Dye eFluor 780 | Invitrogen | 65-0865-14 |

The cells were washed with 4% FBS-PBS. Dead cell staining and surface staining were performed, and after washing, data were acquired using FACSymphony A3 and analyzed using FACSDiva (v8.0.1, BD Biosciences) and FlowJo (TreeStar) software.

### In vitro CAR-T cell survival and function analysis (FIG. 17)

Long-term survival, memory formation, and maintenance of function of each CAR-T cell were evaluated. CAR-T cells were stimulated with stimulants (medium as a negative control, EGFRvIII stimulation beads, or the U87d cell line) at a ratio of 1:1. After 24 hours, the stimulants were removed by ceramic beads or sorting, and the CAR-T cells were divided into 0 mM, 0.5 mM, and 10 mM groups and continuously cultured. Before stimulation and on day 1, day 3, and day 7 after stimulation, together with cell counting, Annexin V/7AAD for evaluating cell survival and cell death, memory phenotypes such as CD27, CCR4, and CD45RA, inhibitory factors such as PD-1, TIM3, and LAG3 (FIG. 19), and others (transcription factors, activation/senescence markers) were analyzed. In addition, RNA was also extracted from each sample, and detailed analysis such as RNA sequencing was also performed. After a series of analyses, some of the day 7 cells were stimulated again in the same groups, and cell survival and function analysis after repeated stimulation was performed in the same manner.

### Cytokine analysis (FCM)

1 × 10⁵ CAR-T cells and 5 × 10⁵ cells of an EGFRvIII-expressing U87 cell line (U87Δ) were co-cultured in 1 ml of medium for 12 hours, and then cultured for 6 hours in the presence of 5 µg/ml Monensin (BD 554724, BD Bioscience), thereby stimulating the CAR-T cells. As the medium, glucose-free RPMI1640 (Wako) supplemented with 10% dialyzed FBS (Cytiva) and adjusted to glucose concentrations of 10 mM and 0.5 mM with a glucose solution (Gibco) was used. Fixation was performed with Fixation/Permeabilization Diluent (Invitrogen); intracellular cytokines were stained with the antibodies shown in Table 1; data were acquired using an LSRFortessa X20; and the data were analyzed using FACSDiva (v8.0.1, BD Biosciences) and FlowJo (TreeStar) software.

### Cytotoxic activity analysis

Target cells (luciferase-expressing U87Δ cells) were seeded in a 96-well plate at 2 × 10⁵ cells per well and co-cultured with CAR-T cells at E : T ratios of 3 : 1, 1 : 1, 0.3 : 1, and 0.1 : 1. After 16 hours, viable cells were caused to luminesce using VivoGlo Luciferin (Promega); luciferase activity was measured using Cytation (Promega); and cytotoxic activity was calculated.

### Metabolic analysis

Metabolic ability of the cells was measured using an XFe24 cell flux analyzer (Bioscience) and an XF Glycolysis Stress Test Kit (Agilent Technologies) according to the product protocol. On the day before analysis, a 24-well flat-bottom plate for analysis was coated with poly-D-lysine (0.1 mg/ml), and a sensor cartridge was hydrated in a CO₂-free incubator. On the day of analysis, the cells for analysis were washed using analysis medium (Seahorse XF RPMI medium, 2 mM L-glutamine), seeded at 2 × 10⁵ cells each in the analysis plate coated with poly-D-lysine, and allowed to stand at 37°C under CO₂-free conditions for 60 minutes. After the sensor cartridge ports were loaded with glucose at a final concentration of 10 mM or 0.5 mM, oligomycin at a final concentration of 1 µM, 2-DG at a final concentration of 50 mM, and, as appropriate, oligomycin and FCCP so as to give final concentrations of 20 µM and 10 µM, respectively, analysis was performed using the flux analyzer, and metabolic function was evaluated based on OCR and ECAR.

### Evaluation of glucose uptake ability

2 × 10⁴ GLUT3 CAR-T cells were seeded in a 96-well plate and cultured in 200 µl of glucose-free RPMI1640 (Wako) for 5 hours. Five minutes before analysis, 1 µl of 2-NDBG (abcam) was added to each well, and after washing, data were acquired using LSRFortessa X20 and analyzed using FACSDiva (v8.0.1, BD Biosciences) and FlowJo (TreeStar) software.

### Intracranial tumor xenograft model

2.5 × 10⁴ cells from the luciferase-expressing U87Δ tumor cell line (U87Δ-luc) were infused intracranially into the right basal cerebrum of NSG mice, and after engraftment, Mock cells, 3C10 CAR-T cells, or GLUT3 CAR-T cells were each administered at 2 × 10⁶ cells via the tail vein. Tumor burden was monitored over time by in vivo imaging, and survival was observed.

### (Methods)

FIG. 1 shows the structure of a metabolically modified CAR (EGFRvIII_CAR_GLUT3). A single-chain antibody (asFv) derived from a mouse-derived antibody (clone 3C10) targeting EGFRvIII expressed in glioblastoma (GBM) was constructed and linked with CD8 hinge, a CD28 transmembrane portion (CD28TM), a CD28 intracellular domain (CD28ICD), 4-1BB ICD, and CD3z to construct a CAR. A high-affinity glucose transporter (GLUT3) was encoded downstream of the CAR via a P2A sequence.

### (Results)

The results are shown in FIG. 2 and subsequent drawings. As shown in FIG. 2, expression of GLUT3 on the T cell surface and enhancement of glucose uptake are shown. A conventional CAR (3C10 CAR) and a metabolically modified CAR (GLUT3 CAR) were introduced into healthy donor-derived T cells by gene transfer to obtain CART cells. Uniform GLUT3 expression was observed on the membrane of GLUT3 CAR-T cells by fluorescence microscopy. In addition, in the GLUT3 CAR-T cells, glucose uptake was enhanced compared with T cells and conventional CAR-T cells (3C10 CAR-T cells).

Next, T cell expansion efficiency comparable to that of conventional CART cells is shown in FIG. 3. As shown in FIG. 3, CAR was genetically introduced after stimulation with anti-CD3/28 beads, and T cell proliferation efficiency was analyzed. GLUT3 CAR-T cells had establishment efficiency comparable to that of conventional CAR-T cells (3C10 CAR-T cells).

Next, enhancement of T cell glycolytic ability by GLUT3 expression is shown in FIG. 4. As shown, metabolism of GLUT3 CAR-T cells and 3C10 CAR-T cells was analyzed using a Flax analyzer. GLUT3 CAR enhanced glycolytic ability in both a low-glucose environment and a normal-glucose environment.

Next, it was shown that GLUT3 CAR-T cells have a competitive advantage under low glucose (FIG. 5). As shown in FIG. 5, 3C10 CAR-T cells and GLUT3 CAR-T cells were co-cultured under normal glucose and under low glucose. Under normal glucose, survival of both was observed to be comparable, whereas under low glucose, significant survival of GLUT3 CAR-T cells was observed, suggesting that GLUT3 CAR-T cells were capable of advantageous glucose uptake.

Next, enhancement of cytokine production ability of GLUT3 CAR-T cells is shown in FIG. 6. As shown in FIG. 6, after antigen stimulation of 3C10 CAR-T cells and GLUT3 CAR-T cells, the proportion of cytokine-producing cells was analyzed by FCM. A significant increase in expression of cytokines (IFN-g, IL-2, TNF-a), which are important for T cell maintenance and activation and exertion of antitumor activity, was observed.

Next, the function of GLUT3 CAR-T cells maintained even under low glucose is shown in FIG. 7. As shown, cytokine production ability under normal glucose and under low glucose was compared. In conventional CAR-T cells (3C10 CAR-T cells), an extreme decrease in function under low glucose conditions was observed. On the other hand, in GLUT3 CAR-T cells, high cytokine production ability was observed under normal glucose conditions, and even under low glucose conditions, GLUT3 CAR-T cells exerted a function comparable to the cytokine production ability of 3C10 CAR-T cells under normal glucose conditions.

Next, enhanced cytotoxic activity of GLUT3 CAR-T cells is shown in FIG. 8. As shown, cytotoxic activity was analyzed using an EGFRvIII-expressing cell line as a target. Cytotoxic activity was analyzed under low glucose conditions and high glucose conditions, and GLUT3 CAR-T cells showed higher cytotoxic activity than 3C10 CAR-T cells under both environments.

Next, properties imparted by GLUT3 CAR to the memory phenotype are shown in FIG. 9. As shown, the memory phenotypes of 3C10 CAR-T cells and GLUT3 CAR-T cells were compared. In GLUT3 CAR-T cells, a tendency toward differentiation into effector T cells was observed.

Next, suppression of inhibitory molecule expression in GLUT3 CAR-T cells is shown in FIG. 10. Expression of inhibitory molecules was compared under unstimulated conditions and under stimulation with EGFRvIII antigen-positive cells. In GLUT3 CAR-T cells, suppression of expression of PD-1, LAG3, and Tim3 was observed.

Next, mRNA analysis of GLUT3 CAR-T cells (evaluation of metabolism, exhaustion, activation, and differentiation) is shown in FIG. 11. Details of 3C10 CART and GLUT3 CAR-T cells were analyzed based on mRNA expression. In GLUT3 CAR-T cells, as with FCM, a decrease in exhaustion-related molecules and increases in lactate metabolism- and glycolysis-related factors were observed. Overall, the data show enhancement of T cell effector functions such as activation and cytokine production.

Next, FIG. 12 shows that the same effect is obtained when GLUT3 is incorporated into CD19 CAR as well as 3C10CAR, that is, universality is demonstrated. GLUT3 was incorporated into CD19 CAR. It was confirmed that an effect similar to that of 3C10 CAR was obtained. In addition, CD19 was expressed in a pancreatic cancer cell line, and it was shown that an effect was also obtained against pancreatic cancer.

As shown in FIG. 13, rapid tumor eradication was achieved in an intracranial xenograft model. Antitumor activity was observed in a U87Δ intracranial xenograft model. In GLUT3 CAR-T cells, more rapid tumor eradication than in 3C10 CAR-T cells was observed. In addition, rejection was confirmed by tumor challenge.

### (Example 2: Enhanced expression of glucose transporter)

In order to achieve enhanced expression of a glucose transporter, a CAR construct that constitutively expresses GLUT3 was designed (stbl-GLUT3 in FIG. 12 = GLUT3 CAR).

Using cells into which the CAR of FIG. 12 was introduced, the function and antitumor effect thereof are analyzed.

FIG. 15 shows the results of evaluating the antitumor effect of GLUT3 CAR-T cells in a similar mouse model. While CR was not obtained with Mock T cells or 3C10 CAR-T cells, CR was obtained in four mice with GLUT3 CAR-T.

FIG. 16 shows the results of tumor challenge. That is, the drawing shows the state of sudden death of mice in the GLUT3 CAR-T cell group. Tumor (U87d) was re-transplanted into cured mice from the experiment in the previous figure, and rejection ability was evaluated ("surrogate for memory formation").

FIG. 17 shows that long-term survival after various stimulations was enhanced. Each CAR-T cell is observed over time under each condition. As a result, under low glucose, conventional CAR-T (3C10) does not exhibit effective cytokine production, whereas GLUT3 CAR-T exhibits effective cytokine production ability. However, due to activation caused by excessive glucose uptake, GLUT3 CAR-T differentiates over time (day 3 or day 7) into CCR7-negative Tem or Temra (terminal differentiation and loss of stemness) and undergoes apoptosis.

In addition, when CAR-T cells (established under normal glucose conditions) are suddenly (day 0) placed under low-glucose conditions of 0 mM or 0.5 mM, GLUT3 CAR-T, which has a high activation state and is highly glucose-dependent, is highly prone to apoptosis.

FIG. 18 shows that long-term survival after stimulation is enhanced. The above antitumor effect, survival, and adverse events are examined in a system similar to that of the mice described above. In Experiment 1, GLUT3 CAR-T shows equivalent antitumor activity. In Experiment 2, apoptosis and over-differentiation occur due to GLUT3 CAR-T overactivation caused by glucose exposure, and antitumor activity is lost. In rechallenge, tumor rejection is achieved with GLUT3 CAR-T cells. In Experiment 4, in proportion to the tumor effect, predominant CAR-T cell engraftment and T-cell infiltration into tumors are observed with GLUT3 CAR-T. In addition, Experiment 3 is an experiment in a liver tumor model in which immunosuppression due to a low-glucose, high-lactic-acid environment has been suggested, and results similar to those in Experiment 2 are expected to be obtained.

FIG. 19 shows data (in vitro data) showing enhancement of long-term survival after stimulation. At each time point on the left, staining data for 7-AAD and Annexin V, which reflect cell death and apoptosis, are shown. Cell death in each CAR-T cell was examined using Annexin V and 7-AAD. In an unstimulated state, differences depending on glucose concentration were observed, but no substantial differences were observed among the CAR-T cells.

### (Example 3: CAR-T Example)

Further demonstration examples using CAR-T are shown below.

### (Materials and methods)

### (Cell lines)

U-87 MG and U-251 MG GBM cell lines were purchased from American Type Culture Collection (ATCC) and Japanese Collection of Research Bioresources Cell Bank, respectively, and cultured in D-MEM (FUJIFILM Wako Pure Chemical Corporation, Osaka, Japan) supplemented with 10% fetal bovine serum (FBS) and 1% penicillin/streptomycin. An EGFRvIII-packaged lentiviral vector was introduced into U-87 MG and U-251 MG cells, and the transduced cell lines were named U-87 MGΔ and U-251 MGΔ, respectively. SUP-T1, NALM6, and AsPC-1 cells were purchased from ATCC and cultured in RPMI1640 (FUJIFILM Wako Pure Chemical Corporation) supplemented with 10% FBS and 1% penicillin/streptomycin. A CD19 truncated vector was introduced into NALM6 cells, and a CD19-expressing AsPC1 cell line was obtained.

### (Flow cytometry assay)

Flow cytometry assays were performed as previously described (40. Kumagai S, Togashi Y, Kamada T, et al. (2020) The PD-1 expression balance between effector and regulatory T cells predicts the clinical efficacy of PD-1 blockade therapies. Nat Immunol; 21(11):1346-58; 41. Kumagai S, Koyama S, Itahashi K, et al. (2022) Lactic acid promotes PD-1 expression in regulatory T cells in highly glycolytic tumor microenvironments. Cancer Cell; 40(2):201-18.e9; 42. Tada Y, Togashi Y, Kotani D, et al. (2018) Targeting VEGFR2 with Ramucirumab strongly impacts effector/activated regulatory T cells and CD8+ T cells in the tumor microenvironment. J Immunother Cancer; 6(1):106.l; and 43. Tanegashima T, Togashi Y, Azuma K, et al. (2019) Immune Suppression by PD-L2 against Spontaneous and Treatment-Related Antitumor Immunity. Clin Cancer Res; 25(15):4808-19). Briefly, cells were washed twice with FACS buffer, and Fc blocking was performed using an Fc receptor blocking solution (BioLegend, San Diego, CA) at 4°C for 10 minutes in the dark. The cells were washed twice with FACS buffer; antibodies targeting cell surface molecules were added; and the cells were incubated at 4°C for 20 minutes in the dark. Thereafter, the cells were washed twice with FACS buffer. For intracellular antigen staining, the cells were incubated in fixation/permeabilization solution (BD Biosciences, San Jose, CA) at room temperature for 1 hour, and then washed twice with wash buffer (BD Biosciences) according to the manufacturer's instructions. Antibodies targeting intracellular antigens were added, and after incubation at 4°C for 20 minutes in the dark, the cells were washed twice. For the apoptosis assay, the cells were washed once with FACS buffer and once with Annexin binding buffer. Thereafter, the cells were stained with Annexin V and 7-AAD at room temperature for 15 minutes. After washing, flow cytometry (FCM) analysis was performed using LSRFortessa X-20 cytometer (BD Biosciences), and analysis was performed using FlowJo ver.10 software (BD Biosciences). Staining solutions were prepared according to the manufacturer's instructions.

### (Cytokine staining)

Intracellular cytokine staining was performed as previously described (40. Kumagai S, et al. (2020) at Immunol; 21(11):1346-58; 41. Kumagai S, et al. (2022) Cancer Cell; 40(2):201-18.e9; 42. Tada Y, et al. (2018) J Immunother Cancer; 6(1):106.l; and 43. Tanegashima T, et al. (2019) Clin Cancer Res; 25(15):4808-19). During the last 5 hours of 6-hour T cell stimulation, monensin was added to the medium to retain cytokines inside the cells. After staining cell surface markers, Cytofix/Cytoperm reagent (BD Biosciences) was added, and the cells were incubated at 4°C for 20 minutes in the dark. Thereafter, the cells were washed twice with wash solution, and antibodies targeting cytokines were added. The cells were incubated at 4°C for 20 minutes in the dark. After washing, FCM analysis was performed using LSRFortessa X-20 cytometer (BD Biosciences), and analysis was performed using FlowJo ver.10 software (BD Biosciences). The antibodies used for cell staining are summarized in Table 2. Antibodies for staining were prepared according to the manufacturer's instructions.

**[Table 2]**

| Reagent (antibody) | Clone | Supplier | Catalog number |
|---|---|---|---|
| Anti-Glucose Transporter GLUT3 + GLUT14 | EPR3915 | Abcam | cat# ab315168 |
| Anti-Glucose Transporter GLUT1 | SPM498 | Abcam | cat#ab40084 |
| Goat Anti-Rabbit IgG H&L Alexa Fluor 488 | Not available | Abcam | cat#ab150077 |
| Goat Anti-Mouse IgG H&L Alexa Fluor 647 | Not available | Abcam | cat#ab150115 |
| Anti-human CD197 (CCR7) PE/Cyanine7 | G043H7 | BioLegend | cat#353226 |
| anti-human CD45RA Alexa Fluor 488 | HI100 | BioLegend | cat#304114 |
| anti-human CD366 (Tim-3) Brilliant Violet 605 | F38-2E2 | BioLegend | cat#345018 |
| Anti-human IFN-γ Alexa Fluor 488 | 4S.B3 | BioLegend | cat#502515 |
| Anti-human CD279 (PD-1) Brilliant Violet 421 | MIH4 | BD | cat#564324 |
| Anti-human CD3 Brilliant Violet 786 | SK7 | BD | cat#563800 |
| Anti-human CD4 APC-R700 | SK3 | BD | cat#566909 |
| Anti-human CD8 Brilliant Violet 521 | SK1 | BD | cat#563919 |
| Anti-human TNF alpha APC | Mab11 | Invitrogen | cat#17-7349-82 |
| Anti-human IL-2 PE/Cy7 | MQ1-17H12 | Invitrogen | cat#25-7029-82 |

### (Killing assay)

A total of 1 × 10⁵ luciferase-expressing cells (U-87 MGΔ, U-251 MGΔ, AsPC-1, and NALM6) were co-cultured with CAR-T cells at the indicated ratios in 200 µL of RPMI 1640 medium (manufactured by FUJIFILM Wako Pure Chemical Corporation) supplemented with low glucose (0.5 mM) or high glucose (10 mM) in flat-bottom 96-well white plates. After 24 hours of culture, Bio-Glo (Promega, Madison, WI) was added to each well, and luminescence was measured using a Cytation5 multiplate reader (Agilent Technologies, Santa Clara, CA). % specific lysis was calculated according to the following formula: % specific lysis= [(experimental lysis - spontaneous lysis)/(maximum lysis - spontaneous lysis)] × 100.

### (Immunofluorescence staining)

After washing, the cells were fixed with 4% paraformaldehyde for 20 minutes at room temperature and permeabilized with cold methanol for 10 minutes at -20°C. The cells were blocked with 3% BSA/PBS for 60 minutes at room temperature. After blocking, the cells were incubated with a primary antibody at 4°C for 2 hours and with a secondary antibody at 4°C for 1 hour. Next, DAPI was added, and the cells were incubated at room temperature for 5 minutes. After washing, the stained cells were fixed onto slides using Cytospin and analyzed using BZ-X710 (KEYENCE Corporation, Osaka, Japan).

### (Immunohistochemical (IHC) staining)

IHC staining was performed on 5 µm-thick sections of formalin-fixed paraffin-embedded specimens. Antigen removal was performed using citrate buffer (pH 6.0) in a steamer, and then, deparaffinization and rehydration were performed. The slides were incubated with a primary antibody for 16 hours and with an HRP-labeled secondary antibody for 1 hour, and then developed with a diaminobenzidine substrate. An anti-CD3ε antibody (clone: SP7, catalog number: ab16669) (Abcam, Waltham, MA) was used for primary staining. The stained slides were counterstained with hematoxylin.

Hematoxylin and eosin (HE) staining was performed according to a standard protocol. The deparaffinized and hydrated slides were placed in hematoxylin solution and incubated for 4 minutes. After washing, the slides were incubated with eosin solution for 2 minutes. The stained slides were scanned at magnifications of 40x and 400x using BZ-X710 (Keyence Corporation). Two pathologists independently evaluated the stained slides.

### (ELISA method)

A total of 2.0 × 10⁵ U-87 MGΔ cells and 2.0 × 10⁵ CAR-T cells were co-cultured in a 24-well plate. After 24 hours of culture, the supernatant was collected and subjected to ELISA to measure cytokine concentrations. ELISA kits for IFN-γ, IL-2, and TNFα (R&D Systems, Minneapolis, MN) were used according to the manufacturer's instructions. Cytokines in mouse serum were analyzed by a high-sensitivity LUMINEX assay according to the manufacturer's instructions (Merck Millipore, Burlington, MA).

### (Production of CAR-T cells)

Peripheral blood was collected from healthy subjects, and peripheral blood mononuclear cells (PBMCs) were isolated by density gradient centrifugation using Ficoll-Paque (GE Healthcare, Chicago, IL). T cells were isolated from PBMCs by negative selection using MojoSort (BioLegend). The isolated T cells were stimulated with anti-CD3/28 beads (Thermo Fisher Scientific, Waltham, MA) at a T cell-to-bead ratio of 1 : 1 (day 0). At 24 hours after stimulation (day 1), the T cells were transduced with CAR lentivirus at an MOI of 3 and cultured in 30 U/ml IL-2. Half of the medium was replaced after 24 hours. After 5 days, the CD3/28 beads were removed. During culture, half of the medium was replaced on days 4 and 10, and the concentration of T cells was adjusted to 0.7 × 10⁶ cells/mL. The developed CAR-T cells were collected on day 10 and subjected to subsequent analysis.

### (Animal model)

Female NSG mice (6 weeks old) were purchased from Jackson Laboratory. Before painful procedures, an anesthetic was intraperitoneally injected to prevent pain. Using a stereotaxic frame, tumor cells were inoculated at a position 2 mm to the right of bregma, 3 mm posterior to bregma, and 3 mm deep from the surface of the brain. A total of 2.0 × 10⁴ cells in 5 mL of PBS were injected over 1 minute, and then the needle was withdrawn by 1 mm and left for an additional 1 minute before being removed. Four days after tumor implantation, tumor growth was confirmed by bioluminescence imaging (BLI), and random assignment to each treatment group was performed. On day 5, 1.0 × 10⁶ CAR-T cells were injected via the tail vein. Tumor growth after CAR-T cell injection was monitored twice weekly by BLI. Animal care and experiments were performed in accordance with the guidelines of the Animal Committee of the National Cancer Center after approval by the Institutional Animal Care and Use Committee of the National Cancer Center.

### (Glucose concentration measurement)

Tissue samples (5 × 5 mm) were washed to remove attached blood. The samples were minced and centrifuged at 4°C for 10 minutes, and tumor interstitial fluid was collected. The glucose concentration in the interstitial fluid was measured using Multiskan GO (Thermo Fisher Scientific) according to the manufacturer's protocol.

### (Metabolic analysis)

OCR (unit: pmol/min) and ECAR (unit: mpH/min) were evaluated using a Seahorse XF-24 metabolic extracellular flux analyzer (Agilent Technologies). CAR-T cells (conv EGFRvIII CAR-T cells, GLUT3 EGFRvIII CAR-T cells, and On-d GLUT3 EGFRvIII CAR-T cells) stimulated with EGFRvIII beads (ACRO Biosystems, Beijing, China) for 24 hours were resuspended in glucose-free unbuffered RPMI-1640 medium (Agilent Technologies) and plated on Seahorse cell plates coated with poly-L-lysine (BD Bioscience) (2.0 × 10⁵ cells per well). Perturbation profiling of metabolic pathway utilization by CAR-T cells was achieved by addition of glucose (10 mM or 0.5 mM), oligomycin (1 µM), and 2-deoxy-D-glucose (50 mM) (all manufactured by Agilent Technologies). Experiments using the Seahorse system were performed under the following assay conditions: mixing for 3 minutes, waiting for 2 minutes, and measurement for 3 minutes. Thereafter, values of metabolic parameters were calculated.

### (RNA sequencing and subsequent analysis)

CAR-T cells (conv EGFRvIII CAR-T cells, GLUT3 EGFRvIII CAR-T cells, and On-d GLUT3 EGFRvIII CAR-T cells) sorted using BD FACSymphony S6 (BD Biosciences) were stimulated with EGFRvIII beads at a ratio of 1:1 under low-glucose (0.5 mM) or high-glucose (10 mM) conditions, supplemented with 30 U/ml IL-2. CAR-T cells were collected on days 3 and 7, and RNA was extracted using an RNeasy kit (QIAGEN, Hilden, Germany) according to the manufacturer's instructions. Complementary DNA (cDNA) was prepared from the isolated RNA using NEBNext Ultra Directional RNA Library Prep Kit (New England BioLabs, Ipswich, MA). RNA-seq libraries were subjected to 150-bp next-generation sequencing from both ends (paired-end reads) using Novaseq X plus (Illumina, San Diego, CA). For expression profiling using RNA-seq data, paired-end reads were aligned to the hg38 human genome assembly by STAR (Dobin A, Gingeras TR (2015) Mapping RNA-seq Reads with STAR. Curr Protoc Bioinformatics; 51:11.4.1-.4.9). RNAseqChef is a web-based platform for systematic transcriptome analysis, gene expression analysis, principal component analysis, and pathway analysis (Etoh K, Nakao M (2023) A web-based integrative transcriptome analysis, RNAseqChef, uncovers the cell/tissue type-dependent action of sulforaphane. J Biol Chem; 299(6):104810).

### (Statistical analysis)

GraphPad Prism 9 (GraphPad Software, San Diego, CA) was used for statistical analysis. Relationships between groups were compared by a t-test or one-way analysis of variance. Survival was analyzed by the Kaplan-Meier method and statistically compared by the log-rank test. A P value of <0.05 was considered statistically significant.

### (Results)

(GBM has low glucose levels in the TME, which impair the function of CAR-T cells.)

Although accumulating evidence indicates that low glucose levels in the TME are a potential barrier to CAR-T cell therapy in solid cancers, the actual glucose concentrations in the TME and how low-glucose conditions affect the function of CAR-T cells have not yet been clarified [Peng JJ, Wang L, Li Z, et al. (2023) Metabolic challenges and interventions in CAR T cell therapy. Sci Immunol; 8(82):eabq3016]. The present inventors examined glucose concentrations in interstitial fluid from surgical specimens and in serum (FIG. 20a). The glucose concentration in the interstitial fluid of GBM specimens was about 10-fold lower than that in serum [less than 0.5 mM (mean: 0.217 mM)] (FIG. 20b). Glucose concentrations are generally low in various types of cancers, such as non-small cell lung cancer and colorectal cancer, but GBM had the lowest glucose concentration in the TME. This indicates that a low glucose concentration in the TME is a characteristic of GBM (FIG. 20c).

Next, the present inventors examined how a low-glucose environment affects functions of CAR-T cells, such as cytokine production. CAR-T cells targeting EGFRvIII, which is a GBM-related antigen (conv EGFRvIII CAR-T cells), were co-cultured with a human GBM cell line expressing EGFRvIII (U-87 MGΔ) under low-glucose conditions (0.5 mM) that reproduced the TME of GBM. Upon stimulation with U-87 MGΔ, production of cytokines (IFN-γ, IL-2, and TNFα) was significantly decreased under the low-glucose environment, even after short-term (16-hour) exposure (FIGS. 20d and 20e). To further examine functional changes in CAR-T cells under low-glucose conditions, CAR-T cells were stimulated for 16 hours under low-glucose (0.5 mM) or high-glucose (10 mM) conditions, and gene expression profiles were analyzed. The gene expression profiles were significantly altered by exposure to the low-glucose conditions (FIG. 22a). Gene sets related to the cell cycle, differentiation, and cytokine production were significantly decreased, whereas gene sets related to cellular responses to glucose starvation and intrinsic apoptosis were increased under the low-glucose conditions (FIGS. 20f and 20g). As described above, the effector functions of CAR-T cells are immediately impaired when exposed to low-glucose conditions such as the TME of GBM.

### (Overexpression of GLUT3 enhances metabolic fitness of CAR-T cells.)

Among the SLC2 family of glucose transporters (GLUTs), GLUT3, which is encoded by SLC2A3, has the highest affinity and is characterized by predominant expression in neurons and glial cells, which have a high demand for glucose [Flavahan WA, Wu Q, Hitomi M, et al. (2013) Brain tumor initiating cells adapt to restricted nutrition through preferential glucose uptake. Nat Neurosci; 16(10):1373-82]. Therefore, the present inventors examined whether expression of GLUT3 by CAR-T cells could promote the activity of CAR-T cells by promoting glucose uptake in the low-glucose TME of GBM. SLC2A3 was fused to a conventional anti-EGFRvIII CAR construct via a self-cleaving P2A sequence, thereby enabling simultaneous stable expression of CAR and GLUT3 (GLUT3 EGFRvIII CAR) (FIG. 21a). GLUT3 was expressed on the membrane (FIG. 21b) of T cells into which GLUT3 EGFRvIII CAR had been introduced (GLUT3 EGFRvIII CAR-T cells), and the expression level thereof was approximately twice that of the parental anti-EGFRvIII CAR-T cells (conv EGFRvIII CAR-T cells) and comparable to that of the GBM cell line U-87 MGΔ, whereas no change was observed in the expression level of GLUT1 (FIGS. 21c and 23a). The amount of glucose uptake measured by a glucose uptake assay using the glucose analog 2-NBDG was greater in GLUT3 EGFRvIII CAR-T cells than in conv EGFRvIII CAR-T cells (FIG. 21d). Accordingly, glycolysis and glycolytic capacity of GLUT3 EGFRvIII CAR-T cells were significantly improved (FIGS. 21e and 23b). Metabolic analysis further confirmed that GLUT3 EGFRvIII CAR-T cells shifted to a more energetic state with high oxygen consumption rate (OCR)^{high} and high extracellular acidification rate (ECAR)^{high}, compared with conv EGFRvIII CAR-T cells (FIG. 21f). In a glucose competition assay in which GLUT3 EGFRvIII CAR-T cells labeled with carboxyfluorescein succinimidyl ester (CFSE) were co-cultured with conv EGFRvIII CAR-T cells at a ratio of 1 : 1, the number of GLUT3 EGFRvIII CAR-T cells relative to conventional cells increased at a low glucose level (0.5 mM) glucose level, but was comparable under high-glucose (10 mM) conditions, suggesting that GLUT3 EGFRvIII CAR-T cells are substantially competitive in terms of glucose uptake, particularly in a low-glucose environment (FIGS. 23c and 23d). Therefore, stable GLUT3 expression by CAR-T cells promotes glucose uptake, makes CAR-T cells competitive in terms of glucose uptake, and enhances metabolic fitness of CAR-T cells in a limited-glucose environment.

Since expression of GLUT3 improved the metabolic fitness of CAR-T cells, the effector function and safety profile thereof were examined. Therefore, the present inventors examined the effector function of GLUT3 EGFRvIII CAR-T cells in both in vitro and in vivo assays. Compared with conv EGFRvIII CAR-T cells, GLUT3 EGFRvIII CAR-T cells significantly increased cytokine production (IFN-γ, IL-2, and TNFα) at both low glucose levels (0.5 mM) and high glucose levels (10 mM) (FIGS. 21g and 21h, and FIG. 24a). Cytokine production was enhanced in both CD8⁺ T cell subsets and CD4⁺ T cell subsets (FIGS. 24b and 24c). GLUT3 EGFRvIII CAR-T cells also showed increased cytotoxicity against U-87 MGΔ cells and U-251 MGΔ cells, another EGFRvIII-expressing GBM cell line, under both low-glucose and high-glucose conditions (FIG. 21i and FIGS. 24d and 24e). Expression of exhaustion-related molecules including PD-1 and Tim-3 after antigen stimulation was higher in GLUT3 EGFRvIII CAR-T cells than in conv EGFRvIII CAR-T cells (FIGS. 21j and 21k, and FIGS. 24f and 24g). In addition, enhancement of effector function by stable GLUT3 expression was similarly observed in CAR-T cells targeting CD19, which is a different antigen (GLUT3 CD19 CAR-T cells). This indicates that enhancement of metabolic fitness by GLUT3 expression is universal in all CARs, regardless of the single-chain variable fragment (scFv) used or the target antigen (FIGS. 25a to 25c). Therefore, stable GLUT3 expression enables CAR-T cells to be activated in a TME with low glucose levels, although an exhausted phenotype is present.

### (Discussion)

Enhancing antitumor effects by improving metabolic fitness of CAR-T cells in the TME is a new concept for accelerating clinical application of CAR-T cell therapy to refractory solid cancers. Some clinical trials of CAR-T cells against GBM have failed to show clinical utility, but immunological monitoring in these clinical trials revealed that abundant CAR-T cells were detected in the TME. [O'Rourke DM, Nasrallah MP, Desai A, et al. (2017) A single dose of peripherally infused EGFRvIII-directed CAR T cells mediates antigen loss and induces adaptive resistance in patients with recurrent glioblastoma. Sci Transl Med; 9(399); Bagley SJ, Logun M, Fraietta JA, et al. (2024) Intrathecal bivalent CAR T cells targeting EGFR and IL13Rα2 in recurrent glioblastoma: phase 1 trial interim results. Nat Med; 30(5):1320-9; Brown CE, Hibbard JC, Alizadeh D, et al. (2024) Locoregional delivery of IL-13Rα2-targeting CAR-T cells in recurrent high-grade glioma: a phase 1 trial. Nat Med; 30(4):1001-12]. Considering the presence of CAR-T cells in the TME, antigen loss and migration failure are excluded as causes of failure, and it can be assumed that specific conditions in the TME of GBM are involved in inducing dysfunction of CAR-T cells. Since effector T cells undergo glycolysis for survival and activation, glucose is an essential nutrient [Ho PC, Bihuniak JD, Macintyre AN, et al. (2015) Phosphoenolpyruvate Is a Metabolic Checkpoint of Antitumor T Cell Responses. Cell; 162(6):1217-28]. Therefore, given that a markedly low-glucose environment is detected in the TME of GBM, low glucose levels in the TME of GBM must be a major metabolic stress for CAR-T cells. Indeed, conv EGFRvIII CAR-T cells are in a dysfunctional state (low cytokine production and high expression of exhaustion markers). Furthermore, because PD-1 signaling disrupts PI3K/Akt/mTOR signaling and the glycolytic pathway is further impaired, effector T cells may enter a negative metabolic cycle [Chang CH, Qiu J, O'Sullivan D, et al. (2015) Metabolic Competition in the Tumor Microenvironment Is a Driver of Cancer Progression. Cell; 162(6):1229-41].

Considering that nervous system cells such as neurons and glial cells, and GBM, particularly GBM expressing EGFRvIII, satisfy their high glucose demand using GLUT3, which is a high-affinity glucose transporter [Vannucci SJ, Maher F, Simpson IA (1997) Glucose transporter proteins in brain: delivery of glucose to neurons and glia. Glia; 21(1):2-21; Chen S, Yang L, Li Z, et al. (2021) EGFR/EGFRvIII partly regulates the tumourigenesis of glioblastoma through the SOX9-GLUT3 axis. Am J Transl Res; 13(6):6055-65; Schmidt S, Hommel A, Gawlik V, et al. (2009) Essential role of glucose transporter GLUT3 for post-implantation embryonic development. J Endocrinol; 200(1):23-33], causing GLUT3 to be highly expressed in CAR-T cells is likely to enable the CAR-T cells to overcome nutrient competition. Indeed, it was demonstrated that the GLUT3 EGFRvIII CAR construct developed by the present inventors enhanced glucose uptake and effector functions such as cytokine production and cytotoxicity in GLUT3 EGFRvIII CAR-T cells.

### (Example 3: Example using precursor cells of other effector T cells)

Similar experiments are performed using other T cells. For example, unpurified peripheral T cells contain Tnv, Tcm, Tem, Temra, and the like, and CAR (for example, constitutive GLUT3 CAR) can be introduced into any of these fractions.

In the present Example, CAR (CAR, constitutive GLUT3 CAR) is introduced into any purified and enriched T cell memory fraction. In another experiment, CAR-T cells enriched with any T cell memory fraction are induced under culture conditions.

### Experimental procedure:

From a T cell source such as peripheral blood, bone marrow, or umbilical cord blood, Tnv, Tscm, and Tcm, which are precursor cells of T_{eff} and are expected to have longer-term survival and maintenance of stemness, or T_{eff} and Temra fractions, which have already differentiated and mainly exert effector functions, are purified and enriched using FACS sorting or magnetic beads based on T cell memory-related surface antigen expression patterns, namely CD45RA⁺CCR7⁺CD95⁻, CD45RA⁺CCR7⁺CD95⁺, CD45RA⁻CCR7⁺, CD45RA⁻CCR7, and CD45RA⁺CCR7⁻, respectively. · Each fraction is stimulated with CD3/28 beads or the like, and CAR is introduced using a lentiviral vector.

In addition, as another method, after gene transfer into bulk T cells, the type and amount of cytokines added are adjusted (by using IL-7 or IL-15, or platelet lysate) to suppress T_{eff} differentiation and induce predominant proliferation of Tnv, Tscm, and Tcm, thereby obtaining precursor cell-dominant CAR-T cells.

In vitro evaluation: These cells are co-cultured with a U87d tumor cell line or EGFRvIII beads, and after 1 day, 3 days, and 7 days, division and proliferation by CFSE staining and cell counting, and activation and exhaustion states by flow cytometry are evaluated.

In vivo evaluation: These cells are administered to the above-described U87d intracranial pharmaceutical model mice, and the antitumor effect is evaluated based on antitumor activity by BLI and survival.

### Results:

Compared with cases in which CAR was introduced into bulk T cells or into more differentiated T_{eff} or Temra, CAR-T cells in which CAR was introduced into precursor cells of T_{eff}, such as Tnv, Tscm, and Tcm, or in which Tnv, Tscm, and Tcm were predominantly expanded, are expected to have high division and proliferation abilities, and long-term division is expected to be observed. In addition, expression of PD1, TIM3, and LAG3 is expected to be low, and the cells are expected to be resistant to exhaustion.

### (Example 4: Example of TCR)

Similar experiments are performed in an example in which TCR is introduced instead of CAR. For example, TCR can also be introduced into fractions such as T_{eff}, Tnv, Tcm, Tem, and Temra.

In the present Example, TCR (constitutive GLUT3TCR) is introduced into any purified and enriched T cell memory fraction. In another experiment, TCR-T cells enriched with any T cell memory fraction are induced under culture conditions.

Various in vitro and in vivo evaluations shown in Examples 1 to 3 are performed.

### (Example 5: Prevention of bacterial infection, prevention of reinfection, and treatment of bacterial infection)

In the present Example, prevention of bacterial infection, prevention of reinfection, and/or treatment of bacterial infection are verified.

### (Methods)

For prevention against bacterial infection (for example, bacterial conjunctivitis), the T cells of the present disclosure modified with an infection-causing antigen of the bacterial infection are administered to a subject not having the bacterial infection.

For prevention of recurrence and/or treatment of bacterial infection (for example, bacterial conjunctivitis), the T cells of the present disclosure are administered to a subject who had and/or has the bacterial infection.

After administration, prognostic follow-up for bacterial infection in the subject is performed by a technique commonly used in the art.

### (Example 6: Prevention of parasitic infection, prevention of reinfection, and treatment of parasitic infection)

In the present Example, prevention of parasitic infection, prevention of reinfection, and/or treatment of parasitic infection are verified.

### (Methods)

For prevention against parasitic infection (for example, Acanthamoeba keratitis), the T cells of the present disclosure modified with an infection-causing antigen of the parasitic infection are administered to a subject not having the parasitic infection.

For prevention of recurrence and/or treatment of parasitic infection (for example, Acanthamoeba keratitis), the T cells of the present disclosure are administered to a subject who had and/or has the parasitic infection.

After administration, prognostic follow-up for parasitic infection in the subject is performed by a technique commonly used in the art.

### (Example 7: Prevention of viral infection, prevention of onset, prevention of reinfection, and treatment of viral infection)

In the present Example, prevention of viral infection, prevention of onset, prevention of reinfection, and/or treatment of viral infection are verified.

### (Methods)

For prevention against viral infection (for example, tuberculosis, malaria, yellow fever virus, smallpox virus, vaccinia, measles/rubella, polio, epidemic parotitis (mumps)/MUMPS, rotavirus infection, varicella (chickenpox), yellow fever, Ebola, West Nile fever, Hib infection, pneumococcal infection, pertussis, Japanese encephalitis, meningococcal infection, salmonella infection, pathogenic E. coli, toxoplasma, zika virus, herpesvirus type 1, EBV/Epstein-Barr virus (herpesvirus type 4), CMV/cytomegalovirus (herpesvirus type 5), influenza (virus), MARS, rabies, diphtheria, and the like, including but not limited thereto), the T cells of the present disclosure modified with an infection-causing antigen of the viral infection are administered to a subject not having the viral infection.

For prevention of recurrence and/or treatment of the above viral infection, the T cells of the present disclosure are administered to a subject who had and/or has the viral infection.

After administration, prognostic follow-up for viral infection in the subject is performed by a technique commonly used in the art.

### (Example 8: Prevention of allergy, prevention of recurrence, and treatment of allergy)

In the present Example, prevention of allergy, prevention of recurrence, and/or treatment of allergy are verified.

### (Methods)

For prevention against allergy (for example, allergic conjunctivitis), the T cells of the present disclosure modified with an allergy infection-causing antigen are administered to a subject not having a causative factor antigen of the allergy.

For prevention of recurrence and/or treatment of allergy (for example, allergic conjunctivitis), the T cells of the present disclosure are administered to a subject who had and/or has a causative factor antigen of the allergy.

After administration, prognostic follow-up for allergy in the subject is performed by a technique commonly used in the art.

### (Example 9: Prevention of autoimmune disease, prevention of recurrence, and treatment of autoimmune disease)

In the present Example, prevention of autoimmune disease, prevention of recurrence, and/or treatment of autoimmune disease are verified.

### (Methods)

For prevention against autoimmune disease (for example, autoimmune uveitis), the T cells of the present disclosure modified with an autoimmune disease infection-causing antigen are administered to a subject not having a causative factor antigen of allergy.

For prevention of recurrence and/or treatment of autoimmune disease (for example, autoimmune uveitis), the T cells of the present disclosure are administered to a subject who had and/or has a causative factor antigen of the autoimmune disease.

After administration, follow-up observation of the prognosis of the autoimmune disease in the subject is performed by a technique commonly used in the art.

### (Note)

Although the present disclosure has been illustrated using preferred embodiments of the present disclosure as described above, it is understood that the scope of the present disclosure should be interpreted only by the claims. It is understood that the patents, patent applications, and other documents cited in the present specification are to be incorporated by reference in the present specification in the same manner as the contents are specifically described in the present specification. The present application claims priority to Japanese Patent Application No. 2024-001532 filed with the Japan Patent Office on January 9, 2024, the entire contents of which are incorporated herein by reference.

### Industrial Applicability

According to the present disclosure, it is possible to provide cells that are competitive with cancer cells even in a glucose-depleted environment such as a tumor microenvironment (TME), without becoming starved or exhausted. Therefore, a strategy for improving the effects of cell-based immunotherapy can be developed, and application in the medical field is expected.

## Claims

1. A T cell with enhanced glucose uptake ability.

2. The T cell according to claim 1, wherein the T cell comprises a chimeric antigen receptor (CAR).

3. The T cell according to claim 1 or 2, wherein the T cell has an effector function.

4. The T cell according to claim 1 or 2, wherein the T cell is a precursor cell of an effector T cell (T_{eff}).

5. The T cell according to any one of claims 2 to 4, wherein the CAR is expressed in the T cell.

6. The T cell according to any one of claims 1 to 5, wherein the glucose transporter comprises GLUT1, GLUT2, GLUT3, and GLUT4.

7. The T cell according to any one of claims 1 to 6, wherein the glucose transporter is GLUT3.

8. The T cell according to any one of claims 1 to 7, wherein the T cell is a human T cell.

9. An effector T cell comprising a chimeric antigen receptor (CAR), the effector T cell being modified to express a glucose transporter and/or having enhanced expression of a glucose transporter, wherein the T cell exhibits an effector function under low-glucose conditions (0.5 mM) that is at least equivalent to that under normal-glucose conditions (10 mM).

10. The T cell according to claim 9, wherein the CAR is expressed in the T cell.

11. The T cell according to claim 9 or 10, wherein the glucose transporter comprises GLUT1, GLUT2, GLUT3, and GLUT4.

12. An effector T cell into which a GLUT3 gene is introduced.

13. The T cell according to any one of claims 9 to 12, wherein the T cell is a human T cell.

14. A cell population comprising T cells with enhanced glucose uptake ability, wherein the T cells comprise cells having a property of exhibiting an effector function when introduced into a body.

15. The cell population according to claim 14, wherein the T cells are modified to express a glucose transporter and/or have enhanced expression of a glucose transporter.

16. The cell population according to claim 14 or 15, wherein the T cells have an effector function.

17. The cell population according to any one of claims 14 to 16, wherein the T cell comprises a chimeric antigen receptor (CAR).

18. The cell population according to any one of claims 14 to 17, wherein the cell population comprises T_{eff}.

19. The cell population according to any one of claims 14 to 18, wherein the cell population comprises precursor cells of T_{eff}.

20. The cell population according to any one of claims 17 to 19, wherein the CAR is expressed in the T cells.

21. The cell population according to any one of claims 14 to 20, wherein the glucose transporter comprises GLUT1, GLUT2, GLUT3, and GLUT4.

22. The cell population according to any one of claims 14 to 21, wherein the glucose transporter is GLUT3.

23. The cell population according to any one of claims 14 to 22, wherein the T cells are human T cells.

24. A cell population comprising effector T cells that comprise a chimeric antigen receptor (CAR) and are modified to express a glucose transporter and/or have enhanced expression of a glucose transporter, wherein the T cells exhibit an effector function under low-glucose conditions (0.5 mM) that is at least equivalent to that under normal-glucose conditions (10 mM).

25. The cell population according to claim 24, wherein the CAR is expressed in the T cells.

26. The cell population according to claim 24 or 25, wherein the glucose transporter comprises GLUT1, GLUT2, GLUT3, and GLUT4.

27. A cell population comprising effector T cells into which a GLUT3 gene is introduced.

28. The cell population according to any one of claims 24 to 27, wherein the T cells are human T cells.

29. A pharmaceutical composition comprising the T cell according to any one of claims 1 to 13 or the cell population according to any one of claims 14 to 28.

30. The pharmaceutical composition according to claim 29, wherein the pharmaceutical composition is for treating or preventing cancer.

31. The pharmaceutical composition according to claim 29 or 30, wherein the pharmaceutical composition is for curing cancer.

32. The pharmaceutical composition according to any one of claims 29 to 31, wherein curing of the cancer is confirmed by tumor eradication.

33. The pharmaceutical composition according to any one of claims 29 to 32, wherein the pharmaceutical composition is for exerting a long-term effect against cancer.

34. The pharmaceutical composition according to any one of claims 29 to 33, wherein the pharmaceutical composition is for prevention of cancer metastasis or prevention of cancer recurrence.

35. The pharmaceutical composition according to any one of claims 29 to 34, wherein the pharmaceutical composition is for preventing recurrence of cancer.
